# EUROPEAN PATENT APPLICATION

(11) **EP 2 487 241 A1**
(43) Date of publication of application: **15.08.2012**
(21) Application number: 11001044.4
(22) Date of filing: 09.02.2011
(51) Int. Cl.: C12N 15/113, A61K 31/713

(54) **RNAi agents and compositions useful as carp therapeutics**

(71) Applicant: Westfälische Wilhelms-Universität Münster, 48149 Münster (DE)
(72) Inventor: Rauch, Gisep, 48151 Münster (DE)
(74) Representative: Lahrtz, Fritz

(57) **Abstract**

The present invention relates to RNAi agents and compositions useful as therapeutics for the treatment and/or prevention of carp diseases and subject-matter related thereto.

SEQUENCE LISTING

<110> Westfälische-Wilhelms-Universität Münster

<120> RNAi agents and compositions useful as carp therapeutics

<130> P67406EP

<160> 16

<170> PatentIn version 3.5

<210> 1
<211> 21
<212> RNA
<213> Artificial Sequence

<220>
<223> antisense strand of siRNA_small21 (without dTdT overhang)

<400> 1
uuaaaguugu ggaacucggc c 21

<210> 2
<211> 21
<212> RNA
<213> Artificial Sequence

<220>
<223> siRNA_heli21 antisense strand (without dTdT overhang and without
phosphorothioate linkages)

<400> 2
ucuagcuuga accucacaca c 21

<210> 3
<211> 25
<212> RNA
<213> Artificial Sequence

<220>
<223> siRNA_poly25 antisense strand (without dTdT overhang)

<400> 3
uaguugaaga caaacagcac gucgc 25

<210> 4
<211> 27
<212> RNA
<213> Artificial Sequence

<220>
<223> siRNA_capsid27 antisense strand (without phosphorothioate
linkages)

<400> 4
ucucucagaa agucguugag guuggug 27

<210> 5
<211> 21
<212> RNA
<213> Artificial Sequence

<220>

<223>  siRNA_small21 sense strand (without dTdT overhang)

<400>  5
ggccgaguuc cacaacuuua a                                                21


<210>  6
<211>  21
<212>  RNA
<213>  Artificial Sequence

<220>
<223>  siRNA_heli21 sense strand (without dTdT and without
       phosphorothioate linkages)

<400>  6
gugugugagg uucaagcuag a                                                21


<210>  7
<211>  25
<212>  RNA
<213>  Artificial Sequence

<220>
<223>  siRNA_poly25 sense strand (without dTdT overhang)

<400>  7
gcgacgugcu guuugucuuc aacua                                            25


<210>  8
<211>  27
<212>  RNA
<213>  Artificial Sequence

<220>
<223>  siRNA_capsid27 sense strand (without phosphorothioate linkages)

<400>  8
caccaaccuc aacgacuuuc ugagaga                                          27


<210>  9
<211>  23
<212>  DNA
<213>  Artificial Sequence

<220>
<223>  siRNA_small21 sense (with overhang)

<400>  9
ggccgaguuc cacaacuuua att                                              23


<210>  10
<211>  23
<212>  DNA
<213>  Artificial Sequence

<220>
<223>  siRNA_small21 (with overhang)

<400>  10
uuaaaguugu ggaacucggc ctt                                              23


<210>  11

<211>  27
<212>  DNA
<213>  Artificial Sequence

<220>
<223>  siRNA_poly25 sense (with overhang)

<400>  11
gcgacgugcu guuugucuuc aacuatt                                          27


<210>  12
<211>  27
<212>  DNA
<213>  Artificial Sequence

<220>
<223>  siRNA_poly25 antisense (with overhang)

<400>  12
uaguugaaga caaacagcac gucgctt                                          27


<210>  13
<211>  1113
<212>  DNA
<213>  Koi Herpes Virus


<220>
<221>  misc_feature
<222>  (1)..(1113)
<223>  >gi|109706605:137928-139040 Koi herpesvirus strain KHV-U,
       complete genome

<400>  13
atgtacggat taaacagcgc gtccggcttt ctcgacacgg aatgggtcga gaagcagggt      60

atggtcaccc cgccagaaga ggtggccaag atgctgaacc ctcacctggc caccatgggc     120

aagcccgtgg acgtgacgag tctgatgctg cccaacggca tctttgtgcc ctacacgccg     180

acgggtcccg agcccaacat cgcaatcggc ggtggttact tttacaggcc ctcttaccac     240

gggtacgggt cggtggccga ggatagcgac gacctgcaga agctgcacaa gaacgtgctg     300

tgtcgcaacg agggacagtg gactcgcacg gccctgatcg gcgtccccac cacgggcaac     360

ttccccgtgg atctatttga ctacaagaac caaaagtacc agatcgtaaa cacgggcccc     420

gacgcgctgc actctggcga cgctttcatg gtggagcctc cgtccgtcga ggcctccaag     480

gcccagatcg acagcttcaa gcgcatcagg gcgcagggca gcttcaggca gcccacgctg     540

ctgggaggcc tgtccgccac caaccgcgtg ccctcggagc tcacgcaccg catgaagcac     600

atcatggcca gggacattgg gctgtgcatg gacattacga cgcccgggca aggcggcgtg     660

caagccggtc tggcgcgact gtaccaggcc cccacggagc tgggtcaggc cgtcaagaac     720

tctgtgatgg ccacaacgct ggactgtatg aactccctgg acgtgatctt ctccatgatg     780

cgacgcatcg ccgctctacc tcgcggcgtg ctcgagactc tggccgagtt ccacaacttt     840

aagcacgggg gacccaacgc gacgatgccc gacccggcca tcctgatcga gatctggagg     900

acacccgccg tcgcggacct cttcatggaa tccatggaca tgggcttcca gtccatcgac     960

cggctcaggc actgcaccta cggcacggtg atcaagtcca cagaggccaa cccaacctcc 1020

acccggggag agatcatcta caacgcttac agcggacagt acgacacggg cgacaccttc 1080

gagggcgccg tactctcctt caacggctac tga 1113

<210> 14
<211> 1923
<212> DNA
<213> Koi Herpes Virus

<220>
<221> misc_feature
<222> (1)..(1923)
<223> >gi|109706605:135907-137829 Koi herpesvirus strain KHV-U,
complete genome

<400> 14
atggacgccg atgagctcga cttttgttgc gatgagtttg atctgtacgg cgaggagccc 60

agcgaggctt ctcggctggg gtacggggac aaggacgatg atgacgacgg cggtgacgac 120

tgcgacgact ctgctagcgt cgtaccgtcg cctgcctcat ctgtgtcttc cacctcgtcc 180

tcttattctt tcaaggcgcc tcaatccagg ttcaacaggg gaaccaacag gttcaaccct 240

ttcaagaaaa aggtgaagga ggatgaggat gaggatgatg aggtgaagga cgaagaggag 300

acgagggtag aggtagagag caagggagcc ctgagcaaga aggtgaccga tcactacctg 360

gcctacacca gccacctcca gagagtgtgg gcctcagaca tcatggacct gctggcgtcg 420

cctaccaacg ctctcctggc cagggtctgt ctgctggccg ggtgcgccgg cgcgggcaag 480

acgtacgcca tcaacatact gagggaggcc gccggccctt actgcgcggt gacggccagc 540

accctgaacg cggcggggat ccacgcggac tctcgcacca tccacagcta cctgggcttc 600

aacacgacgg agctgctgga cctcaactcg agcgaccagg agtggttcgc cgagtacaag 660

aagcggcaca aagacacgtt cgatatgctg cagcaggcgc acaaggccta catcgcgaag 720

cccaaccacg actgtccctc tctccgcatg aggtgcgcca agtgtcaggc ctggatgtcc 780

gagccggacg accgcggctc cggtttcgac accatccagt cccaggcctt cacgggcccc 840

gccaggctgc cgctgctgat catcgacgag tacgggatgc tcacgggcgg catgctggcc 900

cgcgtgctga tcgcgctcaa gctgtgggcc ccgccgggcg accagcacct ggtggtgctc 960

gtgggctccg tcacccagct ccagcccgga cgaggcgtcg aggacaggga ccgtcctgac 1020

gacggcctgt ggacgttcgg caagtgggac caggtgacgg cgtcgcgctt caacctgccc 1080

tttagcgtgc ggtccataga ggactcggag tacagcgagt gcctggacat gctgcagctg 1140

aacgtgagca cggtgcgctt tcaggagatc atgcagtcgc gggtcaacaa ctgcgacagg 1200

ttcgggcccc atacgccgag ggtgatgcac caggacaacg cggtgcgaca gctcaacgac 1260

gcctcgctca agaacacgga cggcgagcag cgcaccttca tgcccaaggt gaaccacaac 1320

aacgtgcaga accccgtcga gcgcaaaaag tttttcgccg tggtgcggca gcgcttcaag 1380

cacatcaact ttttcgacgg ggtggtggtg aaggtgggct cgctggtgtg cgtgctcaag 1440

taccagccgc agaactttga gggctgcctc ggtgtggtgg aagaggtctc gcccgccgtg 1500

cgggtgcgct cgctcgagtc tggcaacgtg cacaacattg gcgtgtgcgt gttcaacttt 1560

gacaggatga ggacggtgga gctgatgccc ctgaagctgg cgcacggcat gaacacttac 1620

ttttgccagg gcctcacgtt caagtttcca gtcgtctacg cgccgcctgc ctactacgcc 1680

atgagcccca tcaagcccag ctgctacgtg gtgtgcacgc gtgtgacaaa tcgcggttta 1740

ctgaacctga ccaattgttc attcgcaaac acgccgacgg gccagacttg ctatttttcc 1800

ccgccgtgtg tgaggttcaa gctagacagc gagctgggct accgactgga caaaaacgca 1860

gtctttaacg acgccccaga tccctctcag agcagcaagt ttaaaaagaa ttggagacgg 1920

tag 1923

<210> 15
<211> 5130
<212> DNA
<213> Koi Herpes Virus

<220>
<221> misc_feature
<222> (1)..(5130)
<223> >gi|109706605:144730-149859 Koi herpesvirus strain KHV-U,
complete genome

<400> 15
atgcaagcct ttaagctgta cgaggccttc agcgactacc tgcccaagca ggtgacggag 60

caagagtttg acaggggcga cggagacaat gagctcatgt acgtcgcctc cgtgaggttg 120

atcgaggagt ccttcagggt ttgggcctgc tggccggact ctggcagaaa gaaatgtttc 180

tggatcaggc ccgtgtggtg gccctgcgtg atgtcggaca gggtgtacga gctcgtgggc 240

aggagcaagc ccttcgcgct ggcctcgacg cggtcgctgc cgcaggtcgg cggcgtgtgg 300

ctcaacaacg ccgacaccaa gcaggacgtg cggtgccggc tgatcctctt caagaacaag 360

agggagcgtc aggtcttcat caacctgctc cgcaaagagt tccccaacga cgccgacgtc 420

ggcaccttcg agggctcaga ctctgacatg atggggatgg ccgagtgcat cctcaacagg 480

ttcctctgct gccactttta cagatctccc gggttcgtct cggacccggc ctcagacaac 540

cccgtcatct cgctgaacga gctgtggtcg tgcgacgccg ccgacgagtc cctctacaac 600

gtgcacttta actttaggga cgaggtgctc ctcaggaagc tgaaggaggc cggggccctg 660

ggcaggcaag aggaggcgcg ggagctggag gccaagcggg cccaggccgt ggcgacgatg 720

ccgcgccggt tggtgcccga gctcgtgtac aggtgcgcct tcttcgacat cgagtgcgtg 780

ttcgactctg accacctgga tcccaacctg gacgtcggcc tggacccgac gttcgccgcc 840

gacctcaaga tcgaagcccg agtgaagtcc tgcttcatca aggactttct ccctcccagg 900

aacacgagca acctcagggg ctacaaagaa gtcacgagcg tgagcctggt gtacggaggc 960

aacgagaggg accaggccac gggccgcaag gtcgagaaga agatcaggga ggtctggtac 1020

aacgccgcga gagtgcaggg ccccggtccc atcaaggacg actcttgcgg cgacgtcgtc 1080

accctcaacg gcgtgctgac ctcctgcgcc agcgagctgg agatgctggt gaggttcatg 1140
agggccgtga gggatcactg cgacgtgctg tttgtcttca actatgattt cgacgtgatg 1200
gtcatcacct cccgagtcaa cttttacagg agcgtgtacc cggaggatcc cctgacctcc 1260
gagctggtgg agctcttcga gcaggccttc tccaaggatc cccgcatggt gcccgccgac 1320
tttacctttc tggacaacaa gcacaacgcc tgctactctg agttgctgga caccatcgac 1380
aagcacaagg aagggttctt tgcggcgtgc aggaaagccg gcgcggcaag ggacgagcgg 1440
ggctgcctgg tgattacccg cacgcacgtg agcgacttct tcccctacca gacggcctgg 1500
aaaaagttta tggataacaa gcagctcaac tcgaccatga ggagcctcgg cgtctacatc 1560
gtggacctga tgaaggtcaa caacaccaag tcggtcaaga gcggcgccag caggttcgtc 1620
aagctggaga cggtggccaa caccatcatc agcaagtcga ggcccttcaa gtgtccgcac 1680
aaggctggca agatcaaggg cgtagcgtac cacgagatgg acgccatgtt cttcaaggga 1740
ggcaaggacc tctggaagta cctcatgtat aatctggctg actcggagct gctggccagg 1800
atcacccgct tcacccgtcc ccacatcgag tttgtgtgca gggtgagggc caccttcggc 1860
ctggactatg tgagtctggg cagggagaag gtagagttca gcggggccat ggtgcagtct 1920
accaagtcgg tggaggctcc cttgctctac agcaaggtcc gcataggacg cttcgtggcg 1980
actggcaaga acttcgcctc ggtggcgatg ggaggcaagt acgcttccat agactacagg 2040
cgcaatatca aggtgaaagg aggcaaggtg tttcagcccc tgctggggat gacctacacg 2100
ggtccctaca tcggcaccgt ctgcacgtac gactttgcca gcctgtaccc cagcaacatg 2160
tgcgacggcg gtatcagccc cgagtcgatc gtgtcgaaga tggacccctt ctgcctcgag 2220
tacgtgagga acacggtcat gctggactgg aagaagatac ccgctgccag caacatggaa 2280
gagatcaggg actacccgca ctcggaggac ctctacacca tcctctgcta caagaacaag 2340
gaggtcggct gggtccgctt cgagacctac acggcgtcta gcctcaacca ctacctgagc 2400
atgaggtccc aatacaagaa gcgcatgaag acggagaagg acgccgggct caaggcctac 2460
tacgaccaga tgcagggcga gatgaaggtg tgcgccaact ctcactacgg agtggcccag 2520
tccctctgcc agcatctcac cacctggtcg ggcaggcaaa agattctgct ggtggagacg 2580
gcggtgaaga agacgccggg catgaccgtc gtgtacggcg acacggacag cgtcatgtac 2640
cagtgtcccc cggccgagat gaaccctctg gacgtggaca ccgtgaggga ggacgtgagc 2700
gccgaagcgg tgaacctcta caccaagggc aagactcggg aggaaggcga gaccgttcag 2760
aggatcctga gggacctcaa cgaccgtctg tacgagttca tggccgagag gatggtgtac 2820
gtcgacgacc aagtgaacgt gaggcctctg tcgcgctgcc ccgagaccaa acacttttac 2880
ctgacggact ctgtggacgg gcagcgggtg tacctcaagg acatctttga ccgcacgctc 2940
atcaccaacc tggcctacga gaacacggct acggtgagca tcaacatggc caagaagaac 3000
tacatctaca ccaaccacga gctggaggac ggggtgctga ccaagaccaa ggagaagctg 3060
aggggggtgc aggccatcaa gaacaacgcc gccggagcga cgcgcgactt caacaacgac 3120

atggtgtacg cttgcttcag gggctgggcg atcgtgtacg ccagcacctt cggcaacatc 3180
aacggcatga tctcgtacaa gagctggggc tcggtgaggg aaggggacga cgtctacttt 3240
tgcacgacgg cgcccgagtt cgacgagtgg cacgcctgca ccaactacga gaagatggtg 3300
tgcgagtggt gcagggtggt gtcgtgcctc acggaacccg cggccagctc ggccgagtac 3360
agcatgcaca ggctcgtgct gaggaagccg gacggcgtcg agttcaagta cgtgtgcgcg 3420
tcggacagta acaacttcaa catgacccac accttctccc tggcggagca gatccgtcgc 3480
gacgccgtgg cgctgaacct gatcaagttc aggtactggt cggcgagcgc tggcttcacg 3540
agctggcgga gcctcatcca gtactctggc ctgcagacgt tcaagcagga gtccctcaag 3600
gtcaccttca acgagtacgg agcggtcaac accaagaaca acaaggtggc ctacgtgacg 3660
gtggagacgc tgtgccgggt ggacgccgag tacaagaagc aggtgcccat cctgagcttt 3720
ctcaagggct tcaggttcga cgtgaagcag aacgtgatgg acagggacat gtggggctcc 3780
tcgagcatcc accttcacga cctgacggtg gacgatctca tggccgcgtt ggagtggccc 3840
gacgtgatca gggacgccga gtggcgcgga cagtcggagg aggccgtcgc ttcggacgag 3900
cggcaggact ctgagcagat gaagctgctg agggacatga aggccgggga gctggcgcgg 3960
gccaaggcca agaaacccaa gagggccaag aagggtacca tggacgcctt cctcacggac 4020
gaagaagaag aaagggtgga ggtgcccccg ctgcgcacga gcggggtgcc cgcggagaac 4080
cacatcgcgc gggacaggcg ggtgcccccg ttccacccga tggccctgaa gcacgctctg 4140
agtctggcgg ccgtcacgga ggacaccaac tacggcgtgc acctgctgtt tgacatgttt 4200
gtgcccaaga cgggcaaggt ccccgtgggg tggcctctga ggaccaagct ggagcgtggc 4260
ctcgcgttcg tgagggacac gtacgaccac atgagattgc tcaaggacaa gggtcaggcc 4320
gtgttcagca tggtgtactc gacgcccgtc aagacggatc tgctggacga cgtcgacttt 4380
gaggagacgt ttgtaaggcc cgtaaacttt ggcgaggaca acgtgatgcg catcctgacg 4440
aggcccgaga tcatgcgggc cctgctggac tctaaccggg agctgctggc caagaagctg 4500
tcctcgaccg tgggcaagtt cacccacgag aacctgaggg tgcccgcctg tctgagggag 4560
ctggtcgggg ccaggctggt gccctacacg ggcgacccta cggacaaaga caccatacaa 4620
ctgtgcgaga atctgtatca gtataggatt ttattgggtg cagcgaaaaa ctattgtaca 4680
gcagagtacg acagacacat acagaaggtg gactggacgc gggcggacct gggcctggag 4740
acgctagagt gtcccccggc cttcgccacg cactttgagc tcaaggtctt ttttaggcgt 4800
cgcaagtctg cgctggacct cttcaagcgg cagtcgtcct ccccccactc ggcggcgtct 4860
tcggtccagt cccaggcctc gccggcgcac tgcgactttt gcggcagata ctggtcgatg 4920
gccctctcgg gtgagaacgc gtccttcgcc aagtttgtgt ctgaccagta cgagccggtc 4980
aggcggctgt cttcctcttg tagtagcggt gcgggtactg cttcgccgcc gtctgtcctg 5040
ctgaggagga aggtcgagcg ggtctgtctc caggagtttt cgcaggtctc tgcgcagcag 5100
caggcctctt gggtttctga gtccgcctga 5130

<210> 16
<211> 3807
<212> DNA
<213> Koi Herpes Virus

<220>
<221> misc_feature
<222> (1)..(3807)
<223> >gi|109706605:168831-172637 Koi herpesvirus strain KHV-U,
complete genome

<400> 16
atggcgagtc aacagtttat catagggcgc cagcccgcca ccatgggcac caactttagc 60

acggacgact ttggcatcaa ccggggctcc gtgaccgacg gcaaccgctt caagcagctg 120

cggtccctta actcgcccgg cgtcaaggac tttatgaaaa agttcaacgg ctggaccggc 180

gagaaactca tcaacagcta cctcgacctg ggcatggagg acatgaagta cgcctggggc 240

gccgacacca acctcaacga ctttctgaga gacggcaagg cgtacgccgt cgctctgggt 300

cgtctgatgg tcggaggctc cctcaccgag ctccagacca agaactacgt gggcctgagc 360

ctcatgtccc gcatgctgct gcccgacggt attcccgaag acaacgccgt cttctccctg 420

tccatcgtct acgaccagat gaccatggcc ctacccggcg ccatccgttc cctcgcagcc 480

gacgtgaacg cctggcagac taccatgtcc ttcggtctca cccagtacac caccagcatc 540

cagttcctgc atcaggagct gctgtacctg gtcaagtccg agcccgtgac cgagcacgcc 600

aggcgcaagt tcggccagat gatgaaccgt ttcaggctga gcatcatcca ctctcacgcg 660

caggccgccg tcaggcagcc agggttcgtc gagaccatca tcggcagaca gctgggcaag 720

tccatcattg acgccgccgg agtcgtgtac aacatcgcga aagccgaggc cctgctgtgc 780

ggcctgtgca cccgtaaccc cgccaccatg acgcccttgg tggagattgc cctgatggcc 840

ctcaccagcc gcggggacct ggacaacagc aacacgcact actttggcgc catgaactcg 900

cagtttttcc tgccccagct ggtcacacac accgccgttg tgagcaccgt aatgcccacc 960

ttcacctaca acaccagccg ggaccgcgtc gaggccgcca agcgctcaga gatcctctcc 1020

gtggcctggg agagcgccgt gatcgctccg ccgggctcgg gccccgtgca gtccgccagc 1080

gccggctaca cagccagcct gacggtgcca gcggcctcca tccccatgga cagcgtgact 1140

ctgcccgccg tcgtggtgag cggaggcgcc gtcgtgccct tcatcctgtt cgacggtcag 1200

cccatctgca gagacgggcc ctccatgggc agaccgtttg tgagcaacgg cctccagtac 1260

agatacttca cagtgggttg cccggctccc atcacctcgg gactgagccc ggccaccctc 1320

ttcgacgaga gcgtgtccaa ccccatcagc gctgccgcgc tgtccatgta caccccacgc 1380

aagacctgcg tgttcgacga gcacggcgac acccgcttcg tcgacctcgc caccctgcac 1440

gtcaaggccg aggtggcaga gctgtgggac atcaacagag cccgccaacg tttcaacgca 1500

ttgtccaccg aacagagggc gacgacgagg gtcgaggcct acctcaacag ggccgacgtg 1560

ctctgggtgg cccccacgca gaagaagctc cagagccagg actgtcccta cgagttctcc 1620

cctcgcttca tgaccagcct ggccctcaac aacggcaacc tgcagtccat cgtcaagcac 1680
aggggagtca tgtggaacca gacggccatg tccgagcccc acatcgagaa catcgccgtc 1740
gcggccgagc tctacaccgc gctgggcctc aacgaccagc agctcaagaa caccgttgcc 1800
ttcatccaaa gctgcggcgc cgccgccgac gctcgcatga tgcgagactg cgtggcactc 1860
aagatgctgg cacacgaggg caccagcctg gacgacctcg agcagcacca cggcaacgcc 1920
gatcccgtcg tggccattct cgacttccac gagcaccgcg ccacccccgt cgccaacacg 1980
ccgagcgcac gcgccgactc cgcgtacgac gtcctgagcg aggtgatccc gcgtctgctc 2040
aacaggctgc tcggagacac gggccacctg ctgctctcgg acccgtgcgg ctggcacatc 2100
tttggccgcg tgctcaactt cttcgagaac cacatcgggg agcgcgacgc caccaacaat 2160
cagcgcatgt cccgctacgg cttccacatg acacacgagc acttctccac gctcatcaag 2220
atcagggaca ccctggccca ctttgaggcc gtcgtcggca agtggctgtc cccgctgcac 2280
ctgcccaacg atccctacgt gttcctcggc gacaacgagc ccatgaccgt cagatggtcc 2340
tggatcgcct gcgccatctt cgacaccgcc gtgctctttg gcacggaggt cggctacctc 2400
aagtcccgcg tcgtggctgg cggcggcggc aaccacttta ggctgcacca cgccacctgc 2460
gccgggtccc tggcctttgt cggcaccaac ctccccaacg tctttgaggg catggtggcc 2520
gcgggcgccc cgaacaggaa catcatgcag ggcgccgtcg ccggcatcga cgtctactcg 2580
cacagcgggg ccaccttcct gtaccagacg gggtacgtcg cccgctcgga ggcagagcac 2640
gacagcgccg tgagctgcct gggacacgcc agggccagcc gtctccgccg gagcttcaac 2700
acctgcccgc tcgtggccct ggccatggcc acccactact cgctggtggt caaccagaag 2760
aacgtcctca actttgacaa gttctactac tcgggatggt cctacctggt ggtgaggagg 2820
ctcgagttcg aggcaggcgg cttctccgtc atgcccgtac agagccacct cttggtcgtg 2880
ggcaacagca agttcgatct ggcccaggag atcggcctcg acggacagtt ctgtctgagg 2940
tccatgatga gatacgccgt cgtgccgttg ggagtgctgg gacagggagt ctacttcccc 3000
gcgtccttca tccgcaacgt caagggactg ggtctcgagt acgtgaacca cctgcagtcc 3060
aagccccgct acagcatgaa ctccttcgac cacaagtcgg cctgcatgca cgccttcatc 3120
atggactggg ccccgtccat gcaccctccc atccagggag cagctaccgg agtgggaccc 3180
atcctgtccg ccaacggtcg cttcaaccct ctcctgggca acgtgagcgc cgccgacttc 3240
ttcaagccgg acgccttcaa cgtgcagcgc ttctacacca cctgcccctt ccagaacccc 3300
tacctgaacg agatgcaggc cgcgctgcac aggatcctcg gccagctcgg cgatcccaac 3360
gcagacatgt tcgcgccggg caagcgcaac gccgtgctca ccaccaccat caagcactac 3420
taccagttcc tcaccgagaa caccagcgtc atgagccgcc tggagaagaa cctccagacc 3480
cgactccaga ccatgggcga ggagtacttt gcgtcccacg gcttcaacaa gagcgccagc 3540
gtcttctacg tggacaacag caccatcggc gcccagagcg aacagctcct caagacgccc 3600
atcgactctg acaaccgcgg caccttcctg atcatgtgca ggacggacaa catcaccttc 3660

gatcccaaga cgaccgacgt gcccatcaac aagctgctgc tggacctcca gggactcaac 3720
ccgctcagac tgctcgaggt caaccccacc aacagagcca acctgaccag gcaaatcctc 3780
aacaacgacc cagcgcacgc cgtttag 3807

## Description

The present invention relates to RNAi agents and compositions useful as therapeutics for the treatment and/or prevention of carp diseases and subject-matter related thereto.

### Background

Common carp (*Cyprinus carpio* L.) is a key source for the production of human nutrition in Europe, Asia and the Middle East. Selective breeding of *C. carpio* to produce special colour morphs, called koi, is in addition an important market segment with a substantial yearly sales volume. Intensive culturing methods for carp production and the frequent and large-scale movement of the ornamental koi have led to a rapid spread of serious diseases (Aoki, T., Hirono, I., Kurokawa, K., Fukuda, H., Nahary, R., Eldar, A., Davison, A.J., Waltzek, T.B., Bercovier, H., Hedrick, R.P., 2007, Genome sequences of three koi herpesvirus isolates representing the expanding distribution of an emerging disease threatening koi and common carp worldwide. Journal of Virology 81, 5058-5065). Koi-herpes virus (KHV known also as Cyprinid herpesvirus 3 or CyHV-3) is thereby one of the most devastating diseases in common carp and koi aquaculture. KHV may cause between 80-100% mortality in susceptible populations and clinical signs most often occur when water temperatures are between 22°C and 27°C. KHV is transmitted via direct body contact, fluids or contaminated water. KHV has been first identified in common carp and koi from Israel, the US, and Germany. It is responsible for mass mortalities in many countries all over the world causing serious economic losses that threaten the livelihood of whole regions.

Contemporary, there is no treatment against KHV able of a fast and effective healing of diseased fish (Hartman, K.H., Yanong, R.P.E., Pouder, D.B., Petty, B.D., Francis-Floyd, R., Riggs, A.C., 2009, Koi Herpesvirus (KHV) Disease. University of Florida IFAS Extension VM-149, 1-9). Ongoing projects refer to vaccines using a live attenuated virus (Patent US 7351527). Other projects base their vaccine research either on an immunogenic peptide (Patent application US 2009/0060951 A1) or on a recombinant alive virus (Patent application WO 2009/027412 A1).

Vaccines have however the important disadvantage that they rely on the up-regulation of the immune system, which may take several weeks in fish (Magnadottir, B., 2006, Innate immunity of fish (overview). Fish & Shellfish Immunology 20, 137-151). As the fish already die two weeks after the outbreak of the disease, the effect of the vaccination will come too late in an acute case. An additional disadvantage of the vaccine is the usage of live viruses, as a back-transformation into a damaging form cannot be ruled out. Furthermore, it is often impossible to discriminate vaccinated fish from infected fish. This distinction is however central for an effective disease control and management.

In one project nucleoside analogues (e.g. A-5021) known to have an inhibitory activity on the human herpes simplex virus (HSV-1), were tested for an anti-KHV effect. However, repeated application of the A-5021 agent did not lead to a significant reduction of the KHV-caused mortality in carp (Patent application WO 2010/012840 A1).

Due to these disadvantages of the vaccine approach and the lack of a fast and effective treatment to cure the devastating KHV-disease, there is a need for new agents and therapies to prevent and/or treat diseases mediated by KHV.

The present invention relates to RNAi agents. RNAi was first discovered in plants and nematodes and its potential to treat viral infections was recognised (Cogoni, C., Macino, G., 2000, Post-transcriptional gene silencing across kingdoms. Current Opinion in Genetics & Development 10, 638-643; Plasterk, R.H.A., Ketting, R.F., 2000, The silence of the genes. Current Opinion in Genetics & Development 10, 562-567; Sharp, P.A., 2001, RNA interference - 2001. Genes & Development 15, 485-490; Sharp, P.A., Zamore, P.D., 2000, Molecular biology - RNA interference. Science 287, 2431-; Zamore, P.D., 2001, RNA interference: listening to the sound of silence. Nature Structural Biology 8, 746-750). RNAi is based on the digestion of long double-stranded RNA (dsRNA), originating for example from RNA viruses, into small pieces of ~21 nt length that are referred to as siRNA (Henschel, A., Buchholz, F., Habermann, B., 2004, DEQOR: a web-based tool for the design and quality control of siRNAs. Nucleic Acids Research 32, W113-W120). These siRNAs can specifically bind to viral mRNAs, leading to the degradation of the viral mRNA (Kim, D.H., Behlke, M.A., Rose, S.D., Chang, M.S., Choi, S., Rossi, J.J., 2005, Synthetic dsRNA Dicer substrates enhance RNAi potency and efficacy. Nature Biotechnology 23, 222-226) and thus to the disruption of the viral replication. The artificial introduction of long dsRNA into mammalian systems is problematic due to an induction of an interferon response which may lead to cell death (Henschel et al., supra). The introduction of short, synthetic siRNA avoids this interferon problem (Kim et al., supra), making it the interesting application in mammal research.

In the present invention, the RNAi-technology was used for the first time to successfully treat the KHV-infection.

### Summary of the Invention

In one embodiment, the invention relates to an RNAi agent, or a composition comprising at least one RNAi agent, or an expression vector capable of transcribing an RNAi agent, or a host cell comprising an expression vector capable of transcribing an RNAi agent for use in prophylactic or therapeutic treatment of freshwater fishes of the family *Cyprinidae,* in particular of the species *Cyprinus carpio* (common carp), most preferably the type Koi.

In a preferred embodiment, *Cyprinus carpio,* in particular Koi are prophylactically or therapeutically treated.

In a further preferred embodiment, the disease is a Koi Herpes Virus (KHV) infection.

In one embodiment, the invention relates to an RNAi agent for use as described above, wherein the antisense strand is complementary to a target RNA transcribed of and/or originating from koi herpes virus (KHV) or contains not more than 1, 2 or 3 nucleotide mismatches to said target RNA within the duplex region.

In a particularly preferred embodiment, the antisense strand of an RNAi agent is complementary or contains not more than 1, 2 or 3 nucleotide mismatches to the RNA molecules transcribed of and/or originating from an KHV ORF with a sequence selected from any of SEQ ID No. 13 to 16 and ORF2 (GenBank ABG42833.1), ORF4 (GenBank ABG42835.1), ORF9 (GenBank ABG42840.1), ORF12 (GenBank ABG42843.1), ORF16 (GenBank ABG42847.1), ORF19 (GenBank ABG42850.1), ORF22 (GenBank ABG42853.1), ORF23 (GenBank ABG42854.1), ORF24 (GenBank ABG42855.1), ORF25 (GenBank ABG42856.1), ORF28 (GenBank ABG42857.1), ORF29 (GenBank ABG42858.1), ORF31 (GenBank ABG42859.1), ORF32 (GenBank ABG42860.1), ORF33 (GenBank ABG42861.1), ORF39 (GenBank ABG42867.1), ORF41 (GenBank ABG42868.1), ORF46 (GenBank ABG42873.1), ORF47 (GenBank ABG42874.1), ORF48 (GenBank ABG42875.1), ORF54 (GenBank ABG42881.1), ORF55 (GenBank ABG42882.1), ORF61 (GenBank ABG42888.1), ORF62 (GenBank ABG42889.1), ORF64 (GenBank ABG42891.1), ORF65 (GenBank ABG42892.1), ORF66 (GenBank ABG42893.1), ORF68 (GenBank ABG42895.1), ORF78 (GenBank ABG42905.1), ORF80 (GenBank ABG42907.1), ORF81 (GenBank ABG42908.1), ORF82 (GenBank ABG42909.1), ORF83 (GenBank ABG42910.1), ORF87 (GenBank ABG42914.1), ORF90 (GenBank ABG42917.1), ORF94 (GenBank ABG42921.1), ORF98 (GenBank ABG42925.1), ORF99 (GenBank ABG42926.1), ORF104 (GenBank ABG42931.1), ORF105 (GenBank ABG42932.1), ORF107 (GenBank ABG42934.1), ORF108 (GenBank ABG42935.1), ORF112 (GenBank ABG42939.1), ORF114 (GenBank ABG42941.1), ORF 115 (GenBank ABG42942.1), ORF116 (GenBank ABG42943.1), ORF 123 (GenBank ABG42950.1), ORF124 (GenBank ABG42951.1), ORF125 (GenBank ABG42952.1), ORF126 (GenBank ABG42953.1), ORF128 (GenBank ABG42955.1), ORF131 (GenBank ABG42958.1), ORF134 (GenBank ABG42961.1), ORF136 (GenBank ABG42963.1), ORF137 (GenBank ABG42964.1), ORF138 (GenBank ABG42965.1), ORF139 (GenBank ABG42966.1) ORF 140 (GenBank ABG42967.1), ORF 141 (GenBank ABG42968.1), ORF 144 (GenBank ABG42971.1), ORF 146 (GenBank ABG42973.1), ORF 148 (GenBank ABG42975.1), ORF149 (GenBank ABG42976.1), ORF 150 (GenBank ABG42977.1), and ORF 153 (GenBank ABG42980.1) within the duplex region.

Preferably the ORF is selected from ORF 72 according to SEQ ID No. 13, ORF 71 according to SEQ ID No. 14, ORF 79 according to SEQ ID No. 15 and ORF 92 according to SEQ ID No. 16 of KHV.

In one embodiment, the invention relates to an RNAi agent wherein the antisense strand is complementary to a target RNA transcribed of and/or originating from koi herpes virus (KHV) or contains not more than 1, 2 or 3 nucleotides mismatches to said target RNA within the duplex region.

In another embodiment, the invention relates to an RNAi agent wherein the antisense strand is complementary to a target RNA transcribed of and/or originating from koi herpes virus (KHV) or contains not more than 1 nucleotide mismatch to said target RNA within the duplex region.

In another embodiment, the invention relates to an RNAi agent wherein the antisense strand is complementary to a target RNA transcribed of and/or originating from koi herpes virus (KHV) or contains not more than 1, 2 or 3 nucleotide mismatches to the RNA molecules transcribed of and/or originating from an ORF selected from ORF 72 according to SEQ ID No. 13, ORF 71 according to SEQ ID No. 14, ORF 79 according to SEQ ID No. 15 and ORF 92 according to SEQ ID No. 16 of KHV within the duplex region.

In a further embodiment, the invention relates to an RNAi agent wherein the antisense strand is at least partially complementary to a target RNA transcribed of and/or originating from the koi herpes virus (KHV).

In another embodiment, the RNAi agent of the invention or for use in the invention is selected from a siRNA or a shRNA molecule.

In another embodiment the invention relates to a composition comprising at least one RNAi agent.

In a further embodiment, the composition comprises 2, 3, 4, 5, 6 or more different RNAi agents.

In one embodiment the RNAi agent antisense strand is complementary within the duplex region to a target RNA transcribed of and/or originating from an open reading frame (ORF) of KHV selected from open reading frames (ORFs) according to SEQ ID No. 13 to 16 and the following ORFs:
ORF2 (GenBank ABG42833.1), ORF4 (GenBank ABG42835.1), ORF9 (GenBank ABG42840.1), ORF12 (GenBank ABG42843.1), ORF16 (GenBank ABG42847.1),
ORF19 (GenBank ABG42850.1), ORF22 (GenBank ABG42853.1), ORF23 (GenBank ABG42854.1), ORF24 (GenBank ABG42855.1), ORF25 (GenBank ABG42856.1),
ORF28 (GenBank ABG42857.1), ORF29 (GenBank ABG42858.1), ORF31 (GenBank ABG42859.1), ORF32 (GenBank ABG42860.1), ORF33 (GenBank ABG42861.1),
ORF39 (GenBank ABG42867.1), ORF41 (GenBank ABG42868.1), ORF46 (GenBank ABG42873.1), ORF47 (GenBank ABG42874.1), ORF48 (GenBank ABG42875.1),
ORF54 (GenBank ABG42881.1), ORF55 (GenBank ABG42882.1), ORF61 (GenBank ABG42888.1), ORF62 (GenBank ABG42889.1), ORF64 (GenBank ABG42891.1),
ORF65 (GenBank ABG42892.1), ORF66 (GenBank ABG42893.1), ORF68 (GenBank ABG42895.1), ORF78 (GenBank ABG42905.1), ORF80 (GenBank ABG42907.1),
ORF81 (GenBank ABG42908.1), ORF82 (GenBank ABG42909.1), ORF83 (GenBank ABG42910.1), ORF87 (GenBank ABG42914.1), ORF90 (GenBank ABG42917.1),
ORF94 (GenBank ABG42921.1), ORF98 (GenBank ABG42925.1), ORF99 (GenBank ABG42926.1), ORF104 (GenBank ABG42931.1), ORF105 (GenBank ABG42932.1),
ORF107 (GenBank ABG42934.1), ORF108 (GenBank ABG42935.1), ORF112 (GenBank ABG42939.1), ORF 114 (GenBank ABG42941.1), ORF 115 (GenBank ABG42942.1),
ORF116 (GenBank ABG42943.1), ORF123 (GenBank ABG42950.1), ORF124 (GenBank ABG42951.1), ORF 125 (GenBank ABG42952.1), ORF126 (GenBank ABG42953.1), ORF128 (GenBank ABG42955.1), ORF131 (GenBank ABG42958.1), ORF134 (GenBank ABG42961.1), ORF136 (GenBank ABG42963.1), ORF137 (GenBank ABG42964.1), ORF138 (GenBank ABG42965.1), ORF139 (GenBank ABG42966.1)
ORF140 (GenBank ABG42967.1), ORF141 (GenBank ABG42968.1), ORF144 (GenBank ABG42971.1), ORF146 (GenBank ABG42973.1), ORF148 (GenBank ABG42975.1), ORF 149 (GenBank ABG42976.1), ORF 150 (GenBank ABG42977.1), and ORF 153 (GenBank ABG42980.1).

In a preferred embodiment, the ORFs are disclosed in Aoki *et al.* (supra).

In a preferred embodiment, the antisense strand of an RNAi agent contains not more than 1, 2 or 3 nucleotides mismatches to said target RNA within the duplex region, most preferred not more than 1 mismatch within the duplex region.

In one embodiment the RNAi agent antisense strand is at least partially complementary to a target RNA transcribed of and/or originating from an open reading frame of KHV selected from open reading frames (ORF) according to SEQ ID No. 13 to 16 and the following ORFs:
ORF2 (GenBank ABG42833.1), ORF4 (GenBank ABG42835.1), ORF9 (GenBank ABG42840.1), ORF12 (GenBank ABG42843.1), ORF16 (GenBank ABG42847.1),
ORF19 (GenBank ABG42850.1), ORF22 (GenBank ABG42853.1), ORF23 (GenBank ABG42854.1), ORF24 (GenBank ABG42855.1), ORF25 (GenBank ABG42856.1),
ORF28 (GenBank ABG42857.1), ORF29 (GenBank ABG42858.1), ORF31 (GenBank ABG42859.1), ORF32 (GenBank ABG42860.1), ORF33 (GenBank ABG42861.1),
ORF39 (GenBank ABG42867.1), ORF41 (GenBank ABG42868.1), ORF46 (GenBank ABG42873.1), ORF47 (GenBank ABG42874.1), ORF48 (GenBank ABG42875.1),
ORF54 (GenBank ABG42881.1), ORF55 (GenBank ABG42882.1), ORF61 (GenBank ABG42888.1), ORF62 (GenBank ABG42889.1), ORF64 (GenBank ABG42891.1),
ORF65 (GenBank ABG42892.1), ORF66 (GenBank ABG42893.1), ORF68 (GenBank ABG42895.1), ORF78 (GenBank ABG42905.1), ORF80 (GenBank ABG42907.1),
ORF81 (GenBank ABG42908.1), ORF82 (GenBank ABG42909.1), ORF83 (GenBank ABG42910.1), ORF87 (GenBank ABG42914.1), ORF90 (GenBank ABG42917.1),
ORF94 (GenBank ABG42921.1), ORF98 (GenBank ABG42925.1), ORF99 (GenBank ABG42926.1), ORF104 (GenBank ABG42931.1), ORF105 (GenBank ABG42932.1),
ORF 107 (GenBank ABG42934.1), ORF108 (GenBank ABG42935.1), ORF 112 (GenBank ABG42939.1), ORF114 (GenBank ABG42941.1), ORF115 (GenBank ABG42942.1),
ORF 116 (GenBank ABG42943.1), ORF 123 (GenBank ABG42950.1), ORF 124 (GenBank ABG42951.1), ORF125 (GenBank ABG42952.1), ORF126 (GenBank ABG42953.1), ORF128 (GenBank ABG42955.1), ORF131 (GenBank ABG42958.1), ORF134 (GenBank ABG42961.1), ORF136 (GenBank ABG42963.1), ORF137 (GenBank ABG42964.1), ORF138 (GenBank ABG42965.1), ORF139 (GenBank ABG42966.1)
ORF 140 (GenBank ABG42967.1), ORF 141 (GenBank ABG42968.1), ORF 144 (GenBank ABG42971.1), ORF146 (GenBank ABG42973.1), ORF148 (GenBank ABG42975.1), ORF149 (GenBank ABG42976.1), ORF150 (GenBank ABG42977.1), and ORF153 (GenBank ABG42980.1).

In a preferred embodiment the open reading frame of KHV is selected from the group consisting of ORF 4, ORF 12, ORF 16, ORF 19, ORF 23 ORF 25, ORF 26, ORF 27, ORF 28, ORF 29, ORF 30, ORF 31, ORF 32, ORF 33, ORF 39, ORF 40, ORF 41, ORF 46, ORF 47, ORF 48, ORF 54, ORF 55, ORF 61, ORF 62, ORF 64, ORF 65, ORF 68, ORF 71, ORF 72, ORF 78, ORF 79, ORF 80, ORF 81, ORF 82, ORF 83, ORF 87, ORF 90, ORF 92, ORF 94, ORF 98, ORF 99, ORF 104, ORF 105, ORF 107, ORF 108, ORF 112, ORF 114, ORF 115, ORF 116, ORF 123, ORF 124, ORF 125, ORF 126, ORF 128, ORF 131, ORF 132, ORF 134, ORF 136, ORF 138, ORF 139, ORF 140, ORF 141, ORF 144, ORF 146, ORF 148, ORF 149, ORF 150, ORF 153 as disclosed in Aoki *et al.* 2007, supra.

In an even more preferred embodiment the ORF is selected from ORF 72 according to SEQ ID No. 13, ORF 71 according to SEQ ID No. 14, ORF 79 according to SEQ ID No. 15 and ORF 92 according to SEQ ID No. 16.

In an embodiment, the open reading frame 72 according to SEQ ID No. 13 refers to capsid triplex protein.

In an embodiment, the open reading frame 71 according to SEQ ID No. 14 refers to a DNA helicase.

In an embodiment, the open reading frame 79 according to SEQ ID No. 15 refers to a DNA polymerase.

In an embodiment, the open reading frame 92 according to SEQ ID No. 16 refers to a major capsid protein.

In a preferred embodiment the genome sequence of KHV is selected from genome sequences according to gene bank entries AP008984, DQ657948, and DQ177346.

In a preferred embodiment, the RNAi agent comprises a sense strand and an antisense strand and wherein the antisense strand comprises a sequence of at least 16, 17, or 18 nucleotides.

In another preferred embodiment, the antisense strand is at least over contiguous 16 or 17 or 18 nucleotides complementary to a target RNA transcribed of and/or originating from an open reading frame of the KHV. In another preferred embodiment, not more than 1, 2, or 3 mismatches occur within the duplex region, most preferably not more than 1 mismatch occurs.

In another preferred embodiment, the RNAi agent comprises a sense strand and an antisense strand, and the sense strand comprises a sequence of at least 16, 17, or 18 nucleotides.

In another preferred embodiment, the sense strand is over at least 16, 17, or 18 contiguous nucleotides complementary to the antisense strand or contains not more than 1, 2, or 3 nucleotide mismatches within the duplex region.

In another embodiment, the invention further refers to an RNAi agent comprising a sense strand and an antisense strand wherein both strands comprise a sequence of at least 16, 17, or 18 nucleotides and/or wherein the antisense strand is at least over contiguous 16 or 17 or 18 nucleotides substantially complementary, preferably complementary to a target RNA transcribed of and/or originating from an open reading frame of the KHV as described in Aoki *et al.* 2007, supra, or wherein not more than 1, 2, or 3 mismatches occur within the duplex region, or most preferably wherein not more than 1 mismatch occurs within the duplex region.

In a further embodiment the sense strand is over at least 16, 17, or 18 contiguous nucleotides substantially complementary, preferably complementary, to the antisense strand or contains not more than 1, 2, or 3 nucleotide mismatches within the duplex region.

In a preferred embodiment, the RNAi agent comprises an antisense strand which is selected from one of SEQ ID NO: 1 to 4, 10, 12 and one of the antisense strands of Table 1.

In another preferred embodiment the invention relates to an RNAi agent wherein the antisense strand comprises at least 15 contiguous nucleic acids of one of SEQ ID NO: 1 to 4, 10, 12 or one of the antisense strands of Table 1.

In a further embodiment 1, 2 or 3 mismatches may be present in the antisense strand compared to a target RNA transcribed of and/or originating from the genomic sequence of KHV within the duplex region.

In a further embodiment, the antisense strand of the RNAi agent is at least partially complementary to the sense strand.

In a preferred embodiment, the antisense strand of the RNAi agent contains not more than 1, 2 or 3 nucleotides mismatches to the sense strand within the duplex region.

In a preferred embodiment, the invention relates to an RNAi agent wherein
(i) the antisense strand comprises at least 15 contiguous nucleic acids of one of SEQ ID NO: 1 to 4 or the molecules of Table (II), or
(ii) the antisense strand is selected from one of SEQ ID NO: 1 to 4 or the molecules of Table (II), or
(iii) the antisense strand of the RNAi agent has at least 80%, preferably at least 90% sequence identity to an antisense strand of (i) or (ii) and/or contains 1, 2 or 3 mismatches to a target RNA transcribed of and/or originating from the genomic sequence of KHV,
wherein Table (II) is:

| Sequence (5'-3') | |
|---|---|
| UUAAAGUUGUGGAACUCGGCCdTdT | (SEQ ID No. 10) |
| UCUAGCUUGAACCUCACACa*c*dT*dT | |
| UAGUUGAAGACAAACAGCACGUCGCdTdT | (SEQ ID No. 12) |
| UCUCUCAGAAAGUCGUUGAGGUUGG*U*G | |

and wherein
(i) * marks phosphorothioate linkages
(ii) the lower case letters indicate 2'-O-methyl-modified nucleotides, and
(iii) dT means deoxythymidine

In a preferred embodiment, the invention relates to an RNAi agent wherein
(a) the RNAi agent is characterized by the sequences selected from SEQ ID No. 1 and SEQ ID No. 5 (siRNA_small21), or SEQ ID No.2 and SEQ ID No.6 (siRNA_heli21), or SEQ ID No.3 and SEQ ID No.7 (siRNA_poly25), or SEQ ID No.4 and SEQ ID No.8 (siRNA_capsid27), or
(b) the RNAi agent is characterized by or comprises the sequences selected from SEQ ID No. 1 and SEQ ID No. 5 (siRNA_small21), or SEQ ID No.2 and SEQ ID No.6 (siRNA_heli21), or SEQ ID No.3 and SEQ ID No.7 (siRNA_poly25), or SEQ ID No.4 and SEQ ID No.8 (siRNA_capsid27), which may optionally carry one or two overhangs and/or which may optionally contain a modified nucleotide and/or nucleotide linkage, or
(c) the RNAi agent is characterized by one or two of the sequences or chemical structures of Table (I):

| Sequence (5'-3') | |
|---|---|
| GGCCGAGUUCCACAACUUUAAdTdT | (SEQ ID No. 9) |
| UUAAAGUUGUGGAACUCGGCCdTdT | (SEQ ID No. 10) |
| GUGUGUGAGGUUCAAGCUAG*A*dT*dT | |
| UCUAGCUUGAACCUCACACa*c*dT*dT | |
| GCGACGUGCUGUUUGUCUUCAACUAdTdT | (SEQ ID No. 11) |
| UAGUUGAAGACAAACAGCACGUCGCdTdT | (SEQ ID No. 12) |
| CACCAACCUCAACGACUUUCUGAGA*G*A | |
| UCUCUCAGAAAGUCGUUGAGGUUGG*U*G | |

Wherein
(i) * marks phosphorothioate linkages
(ii) the lower case letters indicate 2'-O-methyl-modified nucleotides, and
(iii) dT means deoxythymidine

In another preferred embodiment, the invention relates to a composition comprising more than RNAi agent, wherein the more than one RNAi agents are characterized by:
(i) the antisense strand of each RNAi agent comprises at least 15 contiguous nucleic acids of one of SEQ ID NO: 1 to 4 or the molecules of Table (II), or
(ii) the antisense strand of each RNAi agent is selected from one of SEQ ID NO: 1 to 4 or the molecules of Table (II), or
(iii) the antisense strand of each RNAi agent has at least 80%, preferably at least 90% sequence identity to an antisense strand of (i) or (ii) and/or contains 1, 2 or 3 mismatches to a target RNA transcribed of and/or originating from the genomic sequence of KHV,
wherein Table (II) is:

| Sequence (5'-3') | |
|---|---|
| UUAAAGUUGUGGAACUCGGCCdTdT | (SEQ ID No. 10) |
| UCUAGCUUGAACCUCACACa*c*dT*dT | |
| UAGUUGAAGACAAACAGCACGUCGCdTdT | (SEQ ID No. 12) |
| UCUCUCAGAAAGUCGUUGAGGUUGG*U*G | |

and wherein
(i) * marks phosphorothioate linkages
(ii) the lower case letters indicate 2'-O-methyl-modified nucleotides, and
(iii) dT means deoxythymidine

In a preferred embodiment, the composition comprises 2, 3, or 4 different RNAi agents.

In another preferred embodiment, the invention relates to a composition comprising
(a) a mixture of RNA molecules, wherein the RNA molecules are characterized by the sequences according to SEQ ID No. 1 to 8, or
(b) a mixture of RNA molecules, wherein the RNA molecules are characterized by or comprise the sequences according to SEQ ID No. 1 to 8, which may optionally carry one or two overhangs and/or which may optionally contain a modified nucleotide and/or nucleotide linkage, or
(c) a mixture of the molecules, wherein the RNA molecules characterized by the sequences or chemical structures of Table (I):

| Sequence (5'-3') | |
|---|---|
| GGCCGAGUUCCACAACUUUAAdTdT | (SEQ ID No. 9) |
| UUAAAGUUGUGGAACUCGGCCdTdT | (SEQ ID No. 10) |
| GUGUGUGAGGUUCAAGCUAG*A*dT*dT | |
| UCUAGCUUGAACCUCACACa*c*dT*dT | |
| GCGACGUGCUGUUUGUCUUCAACUAdTdT | (SEQ ID No. 11) |
| UAGUUGAAGACAAACAGCACGUCGCdTdT | (SEQ ID No. 12) |
| CACCAACCUCAACGACUUUCUGAGA*G*A | |
| UCUCUCAGAAAGUCGUUGAGGUUGG*U*G | |

Wherein
(i) * marks phosphorothioate linkages
(ii) the lower case letters indicate 2'-O-methyl-modified nucleotides, and
(iii) dT means deoxythymidine

Section (a) above is to be understood, that the mixture comprises 8 RNA molecules, wherein each RNA molecule is characterized by a sequence according to SEQ ID No. 1 to 8, and that all of the SEQ ID No. 1 to 8 are present in such mixture.

Accordingly, Section (b) above is to be understood, that the mixture comprises 8 RNA molecules, wherein each RNA molecule is characterized by or comprise a sequence according to SEQ ID No. 1 to 8, which may optionally carry one or two overhangs and/or which may optionally contain a modified nucleotide and/or nucleotide linkage, and that all of the SEQ ID No. 1 to 8 (optionally modified) are present in such mixture.

Section (c) above is to be understood, that the mixture comprises 8 molecules, wherein each molecule is characterized by a sequence/chemical structure according to Table I, and that all of the molecules according to Table I are present in such mixture.

In one embodiment the RNAi agents are synthetically produced.

In a further embodiment of the present invention, the RNAi agents are produced using an expression vector capable of transcribing RNAi agents *in vivo* and/or *in vitro.*

In a further embodiment, the invention relates to expression vectors capable of transcribing RNAi agents *in vivo* and/or *in vitro.*

In a further embodiment, the invention relates to host cells comprising an expression vectors capable of transcribing RNAi agents *in vivo* and/or *in vitro.*

In a preferred embodiment, the cell is a carp cell, in particular a koi cell.

In a further embodiment, the invention relates to a fish comprising an RNAi agent or composition or expression vector or a cell according to the present invention.

In one embodiment, KHV is selected from of any of strains J, U, and I.

In a further embodiment the virus is a member of the *Alloherpesviridae* family.

In a preferred embodiment, the virus is cyprinid herpes virus 3 (KHV).

In a further embodiment the RNAi agent of the invention is suitable for use in prophylactic and/or therapeutic treatment of freshwater fishes of the family *Cyprinidae* in particular of the species *Cyprinus carpio* (common carp).

In an even more preferred embodiment the fish is the type called koi.

In a preferred embodiment, the disease to be treated is a KHV infection.

In a preferred embodiment, the RNAi agent is derived from, or consists of, or comprises the sense and/or antisense sequences provided according to SEQ ID NO: 1 to 12 and/or Table (I), or at least 10, 11, 12, 13, 14, or 15 contiguous nucleotides thereof.

In a further preferred embodiment such at least 10, 11, 12, 13, 14, or 15 contiguous nucleotides comprise at least 90 % sequence identity to one of the sequences according to SEQ ID NO: 1 to 12 or Table (I).

In a preferred embodiment a mixture of different RNAi agents is used. In a preferred embodiment, the invention relates to a composition comprising such mixture.

In a further embodiment, the composition comprises 2, 3, 4, 5, 6 or more different RNAi agents.

In a preferred embodiment more than one RNAi agents are directed to the same open reading frame in a *Cyprinidae* virus, in particular KHV.

In another preferred embodiment more than one RNAi agents are directed to different open reading frames in a *Cyprinidae* virus, in particular KHV.

In one embodiment, the antisense strand of an RNAi agent is equal to or is at least 14, 15, 16, 17, 18, 19, 25, 29, 40 or 50 nucleotides in length. In a preferred embodiment, the length is equal to or less than 60, 50, 40, 30 nucleotides in length. Preferred ranges are 15 to 30, more preferred 17 to 25, even more preferred 19 to 23 and most preferred 19 to 21 nucleotides in length.

In a further embodiment, the sense strand of an RNAi agent is equal to or is at least 14, 15, 16, 17, 18, 19, 25, 29, 40 or 50 nucleotides in length. In a preferred embodiment, the length is equal to or less than 60, 50, 40, 30 nucleotides in length. In an even more preferred embodiment the range is 15 to 30, even more preferred 17 to 25, 19 to 23 and most preferred 19 to 21 nucleotides in length.

The double-stranded, duplex portion of an RNAi agent preferably equal to or is at least 15, 16, 17, 18, 19, 20, 21, 22, 23, 24, 25, 29, 40, or 50 nucleotide pairs in length. In a preferred embodiment, it is equal to or less than 60, 50, 40, 30 nucleotide pairs in length. In a preferred embodiment the range is 15 to 30, more preferred 17 to 25, even more preferred 19 to 23, most preferred 19 to 21 nucleotide pairs in length.

In another embodiment larger precursor RNAi agents are used which are up to 5000 base pairs in length which are than digested internally to generate the shorter RNAi agent as described above. In a preferred embodiment the precursor RNAi agents are up to 1000 base pairs in lengths and have a minimum length of about 50 base pairs.

In another preferred embodiment the sense and antisense strands are chosen such that the RNAi agent includes a single strand or unpaired region at one or both ends of the molecule. Thus an preferred RNA agent contains sense and anti sense strands preferably paired to contain an overhang, in particular, 1 or 2 5' or 3' prime overhangs, preferably of a length of 1, 2, 3 or 4 nucleotides, more preferably 2 or 3 nucleotides. In a preferred embodiment, the overhang is a 3' overhang.

In an even more preferred embodiment, the unpaired nucleotides forming the overhang are ribonucleotides or deoxyribonucleotides, preferably deoxythymidine (dT), In particular a 2 nt deoxythymidine (dTdT) overhang. 5' prime ends are preferably phosphorylated or they may be unphosphorylated. In another preferred embodiment, the RNAi agents are blunt; i.e. they do not contain overhangs.

In a preferred embodiment, the unpaired nucleotide of the single-stranded overhang that is directly adjacent to the terminal nucleotide pair contains a purine base, and the terminal nucleotide pair is a G-C pair, or at least two of the last four complementary nucleotide pairs are G-C pairs. In further embodiments, the nucleotide overhang may have 1 or 2 unpaired nucleotides, and in an exemplary embodiment the nucleotide overhang is 5'-gc-3'. In preferred embodiments, the nucleotide overhang is on the 3 '-end of the antisense strand. In one embodiment, the RNAi agent includes the motif 5'-cgc-3' on the 3 '-end of the anti sense strand, such that a 2-nt overhang 5'-gc-3' is formed.

Preferred lengths for the duplex region of the antisense and sense strands are between 15 and 30, most preferably 18, 19, 20, 21, 23 or 24 nucleotides in length.

In another embodiment the RNAi agents include modifications (RNAi agents comprising modified RNA) which are for example used to protect the RNAi agents from degradation in biological environments and the improvement of pharmacological properties, e.g. pharmacodynamic properties.

The art has referred to rare or unusual, but naturally occurring, RNAs as modified RNAs, see, e.g., Limbach et al. Nucleic Acids Res. 22: 2183-2196, 1994. Such rare or unusual RNAs, often termed modified RNAs (apparently because they are typically the result of a post-transcriptional modification) are within the term unmodified RNA. Modified RNA refers to a molecule in which one or more of the components of the nucleic acid, namely sugars, bases, and phosphate moieties, are different from that which occurs in nature, preferably different from that which occurs in the human body. While they are referred to as modified "RNAs," they will of course, because of the modification, include molecules which are not RNAs. Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, e.g., non-charged mimics of the ribophosphate backbone.

One or both of the antisense and sense strands of an RNAi agent may be modified. As nucleic acids are polymers of subunits or monomers, many of the modifications described below occur at a position which is repeated within a nucleic acid, e.g., a modification of a base, or a phosphate moiety, or the non-linking oxygen of a phosphate moiety. In some cases the modification will occur at all of the subject positions in the nucleic acid but in many, and in fact in most, cases it will not. By way of example, a modification may only occur at a 3 ' or 5 ' terminal position, may only occur in a terminal region, e.g. at a position on a terminal nucleotide or in the last 2, 3, 4, 5, or 10 nucleotides of a strand. A modification may occur in a double strand region, a single strand region, or in both. E.g., a phosphorothioate modification at a non-linking O-position may only occur at one or both termini, may only occur in a terminal regions, e.g., at a position on a terminal nucleotide or in the last 2, 3, 4, 5, or 10 nucleotides of a strand, or may occur in double strand and single strand regions, particularly at termini. Similarly, a modification may occur on the sense strand, antisense strand, or both. In some cases, the sense and antisense strand will have the same modifications or the same class of modifications, but in other cases the sense and anti sense strand will have different modifications, e.g., in some cases it may be desirable to modify only one strand, e.g. the sense strand.

Other suitable modifications to a sugar, base, or backbone of an RNAi agent are described in PCT/US2004/01193. An RNAi agent can include a non-naturally occurring base, such as the bases described in PCT/US2004/011822. An RNAi agent can include a non-naturally occurring sugar, such as a non-carbohydrate cyclic carrier molecule. Exemplary features of non-naturally occurring sugars for use in RNAi agents are described in PCT/US2004/11829.

An RNAi agent can include an internucleotide linkage, e.g., the chiral phosphorothioate linkage, useful for increasing nuclease resistance. In addition, or in the alternative, an RNAi agent can include a ribose mimic for increased nuclease resistance. Exemplary internucleotide linkages and ribose mimics for increased nuclease resistance are described in. PCT/US2004/07070.

An RNAi agent can include ligand-conjugated monomer subunits and monomers for oligonucleotide synthesis. Exemplary monomers are described in US 10/916,185.

An RNAi agent can have a ZXY structure, such as is described in PCT PCT/US2004/07070.

An RNAi agent can be complexed with an amphipathic moiety. Exemplary amphipathic moieties for use with RNAi agents are described in PCT PCT/US2004/07070.

In another embodiment, the RNAi agent can be complexed to a delivery agent that features a modular complex. The complex can include a carrier agent linked to one or more of (preferably two or more, more preferably all three of): (a) a condensing agent (e.g., an agent capable of attracting, e.g., binding, a nucleic acid, e.g., through ionic or electrostatic interactions); (b) a fusogenic agent (e.g., an agent capable of fusing and/or being transported through a cell membrane); and (c) a targeting group, e.g., a cell or tissue targeting agent, e.g., a lectin, glycoprotein, lipid or protein, e.g., an antibody, that binds to a specified cell type. RNAi agents complexed to a delivery agent are described in PCT/US2004/07070.

An RNAi agent can have non-canonical pairings, such as between the sense and anti sense sequences of the RNAi duplex. Exemplary features of non-canonical RNAi agents are described in PCT/US2004/07070.

One way to increase resistance is to identify cleavage sites and modify such sites to inhibit cleavage, as described in US 60/559,917. For example, the dinucleotides 5'-ua-3', 5'-ca-3', 5'-ug-3', 5'-uu-3', or 5'-cc-3' can serve as cleavage sites. In certain embodiments, all the pyrimidines of an RNAi agent carry a 2'- modification in either the sense strand, the antisense strand, or both strands, and the RNAi agent therefore has enhanced resistance to endonucleases. Enhanced nuclease resistance can also be achieved by modifying the 5 ' nucleotide, resulting, for example, in at least one 5'- uridine-adenine-3 ' (5'-ua-3') dinucleotide wherein the uridine is a 2'-modified nucleotide; at least one 5'-cytidine-adenine-3' (5'-ca-3') dinucleotide, wherein the 5'-cytidine is a 2'- modified nucleotide; at least one 5'-uridine-guanine-3' (5'-ug-3') dinucleotide, wherein the 5'-uridine is a 2'-modified nucleotide; at least one 5'-uridine-uridine-3' (5'-uu-3') dinucleotide, wherein the 5'-uridine is a 2 '-modified nucleotide; or at least one 5'-cytidine- cytidine-3' (5'-cc-3') dinucleotide, wherein the 5'-cytidine is a 2'-modified nucleotide, as described e.g. in PCT/US2005/018931. The RNAi agent can include at least 2, at least 3, at least 4 or at least 5 of such dinucleotides. In a preferred embodiment, the 5' nucleotide in all occurrences of the sequence motifs 5'-ua-3' and 5'-ca-3' in either the sense strand, the anti sense strand, or both strands is a modified nucleotide. Preferably, the 5' nucleotide in all occurrences of the sequence motifs 5'-ua-3', 5'-ca-3' and 5'-ug-3' in either the sense strand, the anti sense strand, or both strands is a modified nucleotide. More preferably, all pyrimidine nucleotides in the sense strand are modified nucleotides, and the 5' nucleotide in all occurrences of the sequence motifs 5'-ua-3' and 5'-ca-3' in the antisense strand are modified nucleotides, or where the anti sense strand does comprise neither of a 5'-ua-3' and a 5'-ca-3' motif, in all occurrences of the sequence motif 5'-ug-3'.

Preferably, the 2'-modified nucleotides include, for example, a 2'-modified ribose unit, e.g., the 2'-hydroxyl group (OH) can be modified or replaced with a number of different "oxy" or "deoxy" substituents.

Examples of "oxy"-2" hydroxyl group modifications include alkoxy or aryloxy (OR, e.g., R = H, alkyl, cycloalkyl, aryl, aralkyl, heteroaryl or sugar); polyethyleneglycols (PEG), O(CH2CH2O)nCH2CH2OR; "locked" nucleic acids (LNA) in which the 2' hydroxyl is connected, e.g., by a methylene bridge, to the 4' carbon of the same ribose sugar; O-AMINE and aminoalkoxy, O(CH2)nAMINE, (e.g., AMINE = NH2; alkylamino, dialkylamino, heterocyclyl amino, arylamino, diaryl 1 amino, heteroaryl amino, or diheteroaryl amino, ethylene diamine, polyamino). It is noteworthy that oligonucleotides containing only the methoxyethyl group (MOE), (OCH2CH2OCH3, a PEG derivative), exhibit nuclease stabilities comparable to those modified with the robust phosphorothioate modification.

"Deoxy" modifications include hydrogen (i.e. deoxyribose sugars, which are of particular relevance to the overhang portions of partially ds RNA); halo (e.g., fluoro); amino (e.g. NH2; alkylamino, dialkylamino, heterocyclyl, arylamino, diaryl amino, heteroaryl amino, diheteroaryl amino, or amino acid); NH(CH2CH2NH)nCH2CH2-AMINE (AMINE = NH2; alkylamino, dialkylamino, heterocyclyl amino, arylamino, diaryl amino, heteroaryl amino, or diheteroaryl amino), -NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino functionality. Preferred substituents are 2'-methoxyethyl, 2'-OCH3, 2'-O-allyl, 2'-C- allyl, and 2'- fluoro.

The inclusion of furanose sugars in the oligonucleotide backbone can also decrease endonucleolytic cleavage. An RNAi agent can be further modified by including a 3' cationic group, or by inverting the nucleoside at the 3'-terminus with a 3'-3' linkage. In another alternative, the 3'-terminus can be blocked with an aminoalkyl group, e.g., a 3' C5-aminoalkyl dT. Other 3' conjugates can inhibit 3 '-5' exonucleolytic cleavage. While not being bound by theory, a 3' conjugate, such as naproxen or ibuprofen, may inhibit exonucleolytic cleavage by sterically blocking the exonuclease from binding to the 3 '-end of oligonucleotide. Even small alkyl chains, aryl groups, or heterocyclic conjugates or modified sugars (D-ribose, deoxyribose, glucose etc.) can block 3'-5'-exonucleases.

Nucleolytic cleavage can also be inhibited by the introduction of phosphate linker modifications, e.g., phosphorothioate linkages. Thus, preferred RNAi agents include nucleotide dimers enriched or pure for a particular chiral form of a modified phosphate group containing a heteroatom at a nonbridging position normally occupied by oxygen. The heteroatom can be S, Se, Nr2, or Br3. When the heteroatom is S, enriched or chirally pure Sp linkage is preferred. Enriched means at least 70, 80, 90, 95, or 99% of the preferred form. Modified phosphate linkages are particularly efficient in inhibiting exonucleolytic cleavage when introduced near the 5'- or 3'-terminal positions, and preferably the 5'-terminal positions, of an RNAi agent.

5' conjugates can also inhibit 5 '-3' exonucleolytic cleavage. While not being bound by theory, a 5' conjugate, such as naproxen or ibuprofen, may inhibit exonucleolytic cleavage by sterically blocking the exonuclease from binding to the 5'-end of oligonucleotide. Even small alkyl chains, aryl groups, or heterocyclic conjugates or modified sugars (D-ribose, deoxyribose, glucose etc.) can block 3'-5'-exonucleases.

In preferred embodiments, RNAi agents are 5 ' phosphorylated or include a phosphoryl analogue at the 5' terminus. 5'-phosphate modifications of the antisense strand include those which are compatible with RISC-mediated gene silencing. Suitable modifications include: 5 '-monophosphate ((HO)2(O)P-O-5'); 5 '-diphosphate ((HO)2(O)P-O- P(HO)(O)-O-5'); 5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O- 5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)2(S)P-O-5'); 5'-monodithiophosphate (phosphorodithioate; (HO)(HS)(S)P-O-5'), 5'-phosphorothiolate ((HO)2(O)P-S-5'); any additional combination of oxygen/sulphur replaced monophosphate, diphosphate and triphosphates (e.g. 5'-alpha-thiotriphosphate, 5'-gamma- thiotriphosphate, etc.), 5'-phosphoramidates ((HO)2(O)P-NH-5\ (HO)(NH2)(O)P-O-5'), 5'- alkylphosphonates (R=alkyl=methyl, ethyl, isopropyl, propyl, etc., e.g. RP(0H)(0)-0-5'-, (OH)2(O)P-5'-CH2-), 5'-alkyletherphosphonates (R=alkylether=methoxymethyl (MeOCH2-), ethoxymethyl, etc., e.g. RP(0H)(0)-0-5'-). The sense strand can be modified in order to inactivate the sense strand and prevent formation of an active RISC, thereby potentially reducing off-target effects. This can be accomplished by a modification which prevents 5 '-phosphorylation of the sense strand, e.g., by modification with a 5'-0-methyl ribonucleotide (see Nykanen et al., (2001) ATP requirements and small interfering RNA structure in the RNA interference pathway. Cell 107, 309-321.) Other modifications which prevent phosphorylation can also be used, e.g., simply substituting the 5'-OH by H rather than O-Me. Alternatively, a large bulky group may be added to the 5'-phosphate turning it into a phosphodiester linkage.

Nitropyrrolyl and nitroindolyl are non-natural nucleobases that are members of a class of compounds known as universal bases. Universal bases are those compounds that can replace any of the four naturally occurring bases without substantially affecting the melting behaviour or activity of the oligonucleotide duplex. In contrast to the stabilizing, hydrogen-bonding interactions associated with naturally occurring nucleobases, it is postulated that oligonucleotide duplexes containing 3 -nitropyrrolyl nucleobases are stabilized solely by stacking interactions. The absence of significant hydrogen-bonding interactions with nitropyrrolyl nucleobases obviates the specificity for a specific complementary base. In addition, various reports confirm that A-, 5- and 6-nitroindolyl display very little specificity for the four natural bases. Interestingly, an oligonucleotide duplex containing 5 -nitroindolyl was more stable than the corresponding oligonucleotides containing 4-nitroindolyl and 6- nitroindolyl. Procedures for the preparation of 1-(2'-O-methyl-β-D-ribofuranosyl)-5- nitroindole are described in Gaubert, G.; Wengel, J. Tetrahedron Letters 2004, 45, 5629. Other universal bases amenable to the present invention include hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, and structural derivatives thereof. For a more detailed discussion, including synthetic procedures, of nitropyrrolyl, nitroindolyl, and other universal bases mentioned above see Vallone et al., Nucleic Acids Research, 27(17):3589-3596 (1999); Loakes et al., J. MoI. Bio., 270:426-436 (1997); Loakes et al., Nucleic Acids Research, 22(20):4039-4043 (1994); Oliver et al., Organic Letters, Vol. 3(13): 1977-1980 (2001); Amosova et al., Nucleic Acids Research, 25(10): 1930-1934 (1997); Loakes et al., Nucleic Acids Research, 29(12):2437-2447 (2001); Bergstrom et al., J. Am. Chem. Soc, 117:1201-1209 (1995); Franchetti et al, Biorg. Med. Chem. Lett. 11:67-69 (2001); and Nair et al, Nucelosides, Nucleotides & Nucleic Acids, 20(4-7):735-738 (2001).

Difluorotolyl is a non-natural nucleobase that functions as a universal base. Difluorotolyl is an isostere of the natural nucleobase thymine. But unlike thymine, difluorotolyl shows no appreciable selectivity for any of the natural bases. Other aromatic compounds that function as universal bases and are amenable to the present invention are 4- fluoro-6-methylbenzimidazole and 4-methylbenzimidazole. In addition, the relatively hydrophobic isocarbostyrilyl derivatives 3 -methyl isocarbostyrilyl, 5 -methyl isocarbostyrilyl, and 3-methyl-7-propynyl isocarbostyrilyl are universal bases which cause only slight destabilization of oligonucleotide duplexes compared to the oligonucleotide sequence containing only natural bases. Other non-natural nucleobases contemplated in the present invention include 7-azaindolyl, 6-methyl-7-azaindolyl, imidizopyridinyl, 9-methylimidizopyridinyl, pyrrolopyrizinyl, isocarbostyrilyl, 7-propynyl isocarbostyrilyl, propynyl-7- azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl, and structural derivates thereof. For a more detailed discussion, including synthetic procedures, of difluorotolyl, 4-fluoro-6-methylbenzimidazole, 4-methylbenzimidazole, and other non-natural bases mentioned above, see: Schweitzer et al., J. Org. Chem., 59:7238-7242 (1994); Berger et al., Nucleic Acids Research, 28(15):2911-2914 (2000); Moran et al., J. Am. Chem. Soc, 119:2056-2057 (1997); Morales et al., J. Am. Chem. Soc, 121 :2323-2324 (1999); Guckian et al., J. Am. Chem. Soc, 118:8182-8183 (1996); Morales et al., J. Am. Chem. Soc, 122(6): 1001-1007 (2000); McMinn et al., J. Am. Chem. Soc, 121 :11585-11586 (1999); Guckian et al., J. Org. Chem., 63:9652-9656 (1998); Moran et al., Proc Natl. Acad. ScL, 94:10506-10511 (1997); Das et al., J. Chem. Soc, Perkin Trans., 1 :197-206 (2002); Shibata et al., J. Chem. Soc, Perkin Trans., 1 :1605-1611 (2001); Wu et al., J. Am. Chem. Soc, 122(32):7621-7632 (2000); O'Neill et al., J. Org. Chem., 67:5869-5875 (2002); Chaudhuri et al., J. Am. Chem. Soc, 117:10434-10442 (1995); and U.S. Patent No. 6,218,108.

In a further embodiment, the invention relates to the RNAi agents, expression vectors, host cells and compositions described above for use in the treatment of KHV infections.

The carp fish, in particular koi, may be treated at various stages of live including sperm stage, unfertilized of fertilized eggs, embryos, immature individuals or mature individuals.

The RNAi agents or compositions according to the present invention may in particular be used to eradicate the virus and/or to reduce the viral load, and/or to reduce the amount of KHV messenger RNA and/or to reduce the disease impact and/or to ameliorate associated disease symptoms of KHV infections and/or to limit the spread of the infectious agent, and/or to destroy the KHV, and/or to incapacitate the virus and/or to prevent the KHV virus from reproducing and/or replicating and/or multiplying and/or render the KHV virus non pathogenic by the treatment.

In another preferred embodiment, the invention relates to the compositions and RNAi agents for use for the prevention of KHV infection and/or the outbreak of the disease mediated by KHV.

In a further embodiment, the invention relates to the compositions and RNAi agents for use in the prophylaxis and or treatment of KHV mediated disease and/or symptoms.

In one embodiment, the treatment refers to the treatment in the acute disease phase.

In another preferred embodiment the composition and agents are for use in the prophylaxis of KHV infection or outbreak.

The methods for delivery may differ and are known to a skilled person include injection or microinjection of RNAi agents, for example intramuscularly or intraperitoneally and/or by feeding RNAi agents and/or expression vectors or cells transformed with an expression vector capable of transcribing RNAi agents of the present invention and/or immersion of the RNAi agent in water and/or transdermal treatment and or treatment of the gills of the fish.

In a further embodiment of the present invention, the RNAi agent and compositions may be delivered in a dry form. In another embodiment they are delivered in aqueous solution. In a further preferred embodiment the RNAi agent and compositions are delivered in a semi-dry form. In one embodiment of the present invention the naked RNAi agent or compositions are transfected. Transfection methods include electroporation, injection, microinjection, jet injection, immersion, ingestion (feeding), calcium phosphate coprecipitation DEAE-dextran mediated transfection, chitosan, nanoparticle formation, usage of aptamers, polybrene-mediated transfection, liposome fusion, lipofection, protoplast fusion, polyethylenimine transfection (soluble and insoluble form), retroviral infection, and biolistics (particle bombardment, e. g. using a gene gun).

In another preferred embodiment, an expression vector capable of expressing RNAi agents according to the present invention are used to transfect the cells.

In another embodiment of the present invention nucleic acid condensing agents are used for transfection such as spermidine, protamine sulphate, poly-lysine, or other agents known in the art.

In a further embodiment of the present invention the agents or compositions of the present inventions are mixed with adjuvants for example Freund's complete adjuvant, Freund's incomplete adjuvant, Saponins (for example QuilA), Muramyl dipeptides, avridine, aluminium hydroxide, aluminium phosphate, oils, oil emulsions, dextran sulphate, glucans, CpG oligomers, artificial RNAs, in particular selected from polyinosinic, polycytidylic, polyadenylic, polyuridylic or polyguanylic acids and combinations thereof, cytokines, and block co-polymers. The adjuvant may be delivered alone or together with the RNAi agents or compositions of the present invention or they are delivered separately thereof.

A particularly preferred adjuvant is an artificial dsRNA or combinations of dsRNAs, in particular selected from combinations of polyinosinic, polycytidylic, polyadenylic, polyuridylic and polyguanylic acids. Particularly preferred are Poly(I:C), Poly(A:U), and Poly(G:C), and combinations thereof. Poly(I:C) is a composition comprising polyinosinic and polycytidylic acids. Poly(A:U) is a composition comprising polyadenylic and polyuridylic acids. Poly(G:C) is a composition comprising polyguanylic and polycytidylic acids.

In a further preferred embodiment, Poly(I:C), or Poly(A:U), or Poly(G:C), or combinations thereof is/are in addition administered separately or together with the compositions or RNAi agents of the present invention. Poly(I:C), or Poly(A:U), or Poly(G:C), or combinations thereof may be administered at the same time point or at different time point(s) as the RNAi agents or compositions of the present invention.

In a preferred embodiment, Poly(I:C) is administered.

In another preferred embodiment, Poly(A:U) is administered.

In another preferred embodiment, Poly(G:C) is administered.

In another preferred embodiment, Poly(I:C) and Poly(A:U) are administered.

In another preferred embodiment, Poly(I:C) and Poly(G:C) are administered.

In another preferred embodiment, Poly(G:C) and Poly(A:U) are administered.

In another preferred embodiment, Poly(I:C), Poly(G:C) and Poly(A:U) are administered.

In one embodiment, the RNAi agents and compositions are delivered as a solution. For such solution, aqueous solvents such as water, saline or PBS, oil, dextrose, glycerol, wetting or emulsifying agents, bulking agents, stabilizers, anti-oxidants, preservatives, coatings, binders, fillers, disintegrants, diluents, lubricants, pH buffering agents, detergents, bile salts, and fusidic derivatives.

An "RNAi agent" as used in the present application is an RNA agent which can downregulate the expression of an open reading frame in a pathogen of *Cyprinidae* fish, or which can downregulate the expression of an open reading frame in a *Cyprinidae* fish.

The pathogen may be a for example a virus, a bacterium, or a eukaryotic pathogen, like a protozoan pathogen or fungus. In a preferred embodiment, the pathogen is a virus, more preferred a KHV virus.

In case the RNAi agent can downregulate the expression of an open reading frame in a *Cyprinidae* fish, such open reading frame is involved in, or mediates the pathogen infection and/or proliferation and/or survival. For example, the *Cyprinidae* fish open reading frame may encode for a membrane receptor protein which is used by a pathogenic *Cyprinidae* fish virus to attach to the host cell membrane.

The RNAi agent is a double stranded RNAi agent which includes more than one, preferably two strands, in which interstrand hybridization can form a region of duplex structure.

A "strand" herein refers to a contiguous sequence of nucleotides, including non-naturally occurring or modified nucleotides. The two or more strands may be or each form part of, separate molecules, or they may be covalently interconnected e.g. by a linker (e.g. a polyethylene glycol linker), to form one molecule. At least one strand can include a region which is sufficiently complementary to a target RNA in a *Cyprinidae*; such strand is termed the "antisense strand". A second strand of the RNAi agent which comprises the region complementary to the antisense strand, is termed the "sense strand".

However, an RNAi agent can also be formed from a single RNA molecule which is at least partly self-complementary forming e.g. a hairpin or pan-handle structure including a duplex region. The latter are herein referred to as short hairpin RNAs or shRNAs. In such case the term "strand" refers to one of the regions of the RNA molecule that is complementary to another region of the same RNA molecule.

The term "mediates RNA" refers to the ability of an agent to silence in a sequence-specific manner a target gene.

"Silencing a target gene" means the process whereby a cell containing and/or expressing a certain product of the target gene, will contain and/or express at least 10 %, 20 %, 30 %, 40 %, 50 %, 60 %, 70 %, 80 %, or 90 % less of such gene product when contacted with the agent as compared to a similar cell which has not been contacted with the agent. Such product of the targeting can for example be a messenger RNA (mRNA), a protein, or a regulatory element. In a preferred embodiment, such product is a protein.

"At least partially complementary" according to the present invention indicate a sufficient degree of complementarity such that stable and specific binding occurs between an RNA agent of the present invention and a target sequence, namely a target RNA molecule from a *Cyprinidae* virus, in particular KHV.

Specific binding requires a sufficient degree of complementarity to avoid non-specific binding of the oligomeric compound to a non target sequence under conditions in which non-specific binding is desired i.e. under physiological conditions in the case of *in vivo* assays or therapeutic or prophylactic treatment or in the case of *in vitro* assays, under conditions in which assays are performed. The non-target sequences typically differ from target sequences by at least 2, 3 or 4 nucleotides.

An RNAi agent is sufficiently complementary to a target RNA that is a target mRNA if the RNAi agent reduces the production of a protein encoded by the target RNA in a cell.

The RNAi agent may be exactly complementary to the target RNA which means that the target RNA and the RNAi agent anneal preferably to form a hybrid made exclusively of Watson-Crick base pairs in the region of exact complementarity.

A sufficiently complementary RNAi agent can include an internal region which is in a preferred embodiment at least 10 nucleotides, that is exactly complementary to a target sequence in a *Cyprinidae* virus open reading frame (ORF).

In another embodiment the RNAi agent specifically discriminates a single nucleotide difference. In such case the RNAi agent only mediates RNAi if exact complementary is found in the region of the single nucleotide difference.

SEQ ID NO: 1 relates to the antisense strand of RNAi agent sRNA_small21, without any modified linkages, dTdT overhangs or modified nucleotides, which is directed to open reading frame 72 of KHV virus.

SEQ ID NO: 2 relates to the antisense strand of RNAi agent siRNA_heli21, without any modified linkages, dTdT overhangs or modified nucleotides, directed to open reading frame 71 of KHV.

SEQ ID NO: 3 relates to the antisense strand of RNAi agent siRNA_poly25, without any modified linkages, dTdT overhangs or modified nucleotides, directed to open reading frame 79 ofKHV.

SEQ ID NO: 4 relates to the antisense strand of RNAi agent siRNA_capsid27, without any modified linkages, dTdT overhangs or modified nucleotides, directed to open reading frame 92 ofKHV.

SEQ ID NO: 5 relates to the sense strand of RNAi agent sRNA_small21, without any modified linkages, dTdT overhangs or modified nucleotides, directed to open reading frame 72 of KHV.

SEQ ID NO: 6 relates to the sense strand of RNAi agent siRNA_heli21, without any modified linkages, dTdT overhangs or modified nucleotides, directed to open reading frame 71 of KHV.

SEQ ID NO: 7 relates to the sense strand of RNAi agent siRNA_poly25, without any modified linkages, dTdT overhangs or modified nucleotides, directed to open reading frame 79 of KHV.

SEQ ID NO: 8 relates to relates to the sense strand of RNAi agent siRNA_capsid27, without any modified linkages, dTdT overhangs or modified nucleotides, directed to open reading frame 92 of KHV.

SEQ ID No. 9 relates to the sense strand of RNAi agent sRNA_small21 (including dTdT overhang).

SEQ ID No. 10 relates to the antisense strand of RNAi agent sRNA_small21 (including dTdT overhang).

SEQ ID No. 11 relates to the sense strand of RNAi agent siRNA_poly25 (including dTdT overhang).

SEQ ID No. 12 relates to the antisense strand of RNAi agent siRNA_poly25 (including dTdT overhang).

An overview of the sequences and molecules is given in Table 1:

**Table 1**

| Name | Strand | Sequence (5'-3') |
|---|---|---|
| siRNA small21 | Sense | GGCCGAGUUCCACAACUUUAAdTdT |
| | Anti-sense | UUAAAGUUGUGGAACUCGGCCdTdT |
| siRNA heli21 | Sense | GUGUGUGAGGUUCAAGCUAG*A*dT*dT |
| | Anti-sense | UCUAGCUUGAACCUCACACa*c*dT*dT |
| siRNA, poly25 | Sense | GCGACGUGCUGUUUGUCUUCAACUAdTdT |
| | Anti-sense | UAGUUGAAGACAAACAGCACGUCGCdTdT |
| siRNA capsid27 | Sense | CACCAACCUCAACGACUUUCUGAGA*G*A |
| | Anti-sense | UCUCUCAGAAAGUCGUUGAGGUUGG*U*G |

| | | |
|---|---|---|
| The (*) marks phosphorothioate linkages and the lower case letters 2'-O-methyl-modified nucleotides. dT means deoxythymidine. | | |

### Figure Legend:

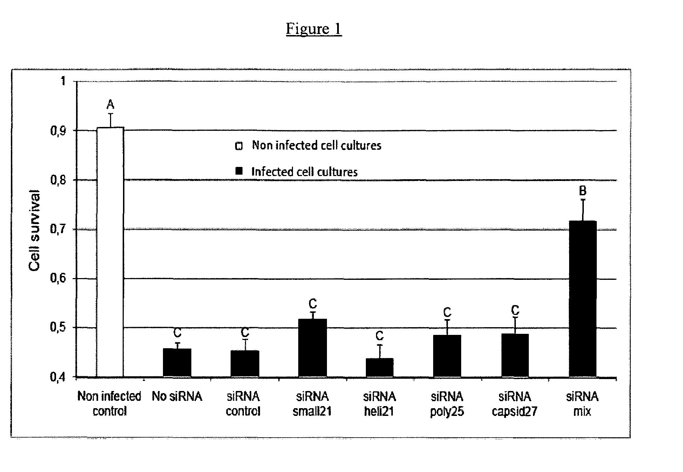

Figure 1 shows the effect of the treatment of KHV infected cell cultures using RNAi agents or compositions according to the present invention. Cell survival of non-infected (white bar) and KHV infected cultures (black bar) are shown. The infected cultures remained either untreated or receive 6 different RNAi treatments. HiPerFect was added to all 8 treatments. Cell survival was quantified with the crystal violet staining method (absorbent at 590 Nanometre with plus minus s.e.). Bars labelled with different letters are significantly different (fisher least significant difference post hoc test: p-level = 0,05).

### Examples

The RNAi agents used in the experiments are siRNAs.

### siRNA design

KHV is a member of the *Alloherpesviridae* family. It has a double-stranded DNA genome with an estimated size of 295 kbp (Aoki et al.,supra). Four siRNAs targeting either the capsid triplex protein, the DNA helicase, the DNA polymerase or the major capsid protein of KHV were designed. The four sequences are listed as ORF 72, ORF 71, ORF 79 and ORF 92 in the KHV Genome project (Aoki et al., supra). For each protein, the sequences of three KHV strains originating from Japan, US and Israel were aligned. GenBank accession numbers of the strains are AP008984 (strain Japan), DQ657948 (strain US) and DQ177346 (strain Israel). The alignment was done using the ClustalW multiple alignment algorithm implemented in BioEdit. The homologous sequence regions of the four proteins were copied into the DEQOR siRNA design program (Henschel et al., supra).

The RNAi agents as listed in Table 1 are either 23 or 27 bp long and with or without a dTdT overhang. The (*) marks phosphorothioate linkages and the lower case letters 2'-O-methyl-modified nucleotides. The siRNAs were purchased from Biomers.net (Germany). As a negative control a commercially available non-silencing siRNA ("control siRNA") from Qiagen was used.

### Cell culture experiments

The common carp brain (CCB) cell-line developed in the Fish Disease Research Unit, University of Veterinary Medicine, Hannover (Neukirch, M., Bottcher, K., Bunnajirakul, S., 1999, Isolation of a virus from koi with altered gills. Bulletin of the European Association of Fish Pathologists 19, 221-222) was used for the *in-vitro* experiments. The CCB cells were grown in Minimum Essential Medium (MEM) with Earle's salts supplemented with 10 % fetal bovine serum, 0,3 % glucose, 1 x non-essential amino acid, 100 U/ml penicillin and 100 µg/ml streptomycin. The KHV virus strain I was used (Israel) (Hedrick, R.P., Gilad, O., Yun, S., Spangenberg, J.V., Marty, G.D., Nordhausen, R.W., Kebus, M.J., Bercovier, H., Eldar, A., 2000, A herpes virus associated with mass mortality of juvenile and adult koi, a strain of common carp. Journal of Aquatic Animal Health 12, 44-57). The virus was inoculated in 4 common carp individuals (one year old) by intraperitoneal injection of 750 TCID50 and subsequently isolated from a fin after 4 days of infection according to a standard protocol using the CCB cell-line (Ganzhorn, J., LaPatra, S.E., 1994, Virology I. General procedures. In J. C. Thoesen, editor. Suggested procedures for the detection and identification of certain fin fish pathogens, 4th edition. American Fisheries Society, Fish Health Section, Bethesda, Maryland; Neukirch et al., supra). The virus was propagated by a single passage in CCB, where cells were mechanically disrupted after occurrence of CPE and the viral suspension was collected after pelleting the cells debris with centrifugation (2600 x g, 30 min. 4°C).

The virus was identified as KHV in the fish tissue with the diagnostic real-time PCR (Gilad, O., Yun, S., Zagmutt-Vergara, F.J., Leutenegger, C.M., Bercovier, H., Hedrick, R.P., 2004, Concentrations of a Koi herpesvirus (KHV) in tissues of experimentally infected Cyprinus carpio koi as assessed by real-time TaqMan PCR. Diseases of Aquatic Organisms 60, 179-187). Further more the presence of the virus in the CCB cells was confirmed with immunocytochemistry assays (Aquatic Diagnostics Anti-Koi Herpesvirus monoclonal antibody P14, Rabbit Anti-Mouse Immunoglobulins polyclonal antibody FITC).

For the RNAi experiments 3 x 10⁵ CCB cells were seeded in 24-well plates in 1 ml of cell culture medium. The 48 h old CCB monolayers (95% confluency) were infected with 100 µl of the viral suspension (300 TCID50). After one hour the virus suspension was removed and 100 µl of the siRNA-solution or a control-solution was added drop-wise to each well. 100µl siRNA-solution contained 1.5 µl of the annealed siRNA (20µM dissolved in HEPES buffer), 95,5 µl cell culture medium (without fetal bovine serum) and 3 µl HiPerFect transfection reagent (Qiagen). For the control-solution, the siRNA was exchanged with cell culture medium. For the non-infected control, we first added 100 µl cell culture medium instead of the virus addition, and one hour later a solution consisting of 97 µl cell culture medium and 3 µl HiPerFect. All solutions were vortexed and incubated at room temperature for 10 minutes before adding to the cell culture.

The following seven treatments were applied to the infected cell cultures: (1) No addition of any siRNA (control-solution), addition of (2) control siRNA from Qiagen, (3) siRNA_small21, (4) siRNA_heli21, (5) siRNA_poly25, (6) siRNA_capsid27 and (7) a mixture of all four siRNAs (siRNA_small21, siRNA_heli21, siRNA_poly25, siRNA_capsid27). The siRNA concentration in the mixture treatment was four times higher compared to the other siRNA treatments. For each treatment, including the non-infected control, we tested three separate wells, resulting in 8x3 = 24 cell culture wells. The cell cultures were incubated after the addition of the siRNA-solution for eight days at 25°C and 2% CO².

### Crystal violet measurement

Cell survival was quantified with the Crystal violet staining method, which is based on the ability of a cell to retain the Crystal violet dye during solvent treatment. Cell monolayers were fixed with formalin in a final concentration of 4 % and the liquid was discarded after 10 minutes. Fixed monolayers were washed with distilled water and the liquid was discarded. The cells were stained for 10 min with 0.5% crystal violet solution in 25% methanol. The crystal violet solution was removed and plates were rinsed with distilled water a few times until colour no longer was coming off in the rinse. The plates were dried at room temperature overnight. The bound dye was extracted with 500 µl of 10 % acetic acid per well, the suspension was transferred on 96 well plate and the optical density of the dye extracts was measured at 590 nm using an automated microtiter plate reader (Fluostar optima). The optical density of the dye is thereby used to quantify the number of surviving cells per well.

### RNAi-therapy in fish

Specific pathogen free, 6-12 months old common carps (10-20g) are purchased from Wageningen University, Netherlands. The fish are briefly anesthetized in a Tricaine solution and half of the fish are injected intraperitoneal with the KHV virus in a dose of 400 TCID50 suspended in 200 µl tissue culture medium. The other half of the fish is injected with 200 µl virus free tissue culture medium. Immediately after this treatment fish are injected intraperitoneally with 100 µl RNAi-solution or control-solutions. The RNAi-solution is composed of 15 µl of the annealed RNAi agent (20 µM dissolved in HEPES buffer), 55 µl of 0.9% NaCl solution and 30 µl HiPerFect. In two control-solutions either the RNAi or the RNAi and HiPerFect are replaced with 0.9 % NaCl. All solutions are vortexed and incubated at room temperature for 10 minutes before injection. To control for an effect of the injection procedure, carp are included which do not receive any injections.

The same six siRNA treatments as in the cell culture experiments are applied, resulting in eight treatments with the two control-solutions. 10 fish per treatment combination are used resulting in 10 fish x 2 (infected/non-infected) x 8 treatments = 160 experimental fish plus 10 fish which do not receive any injection.

Fish are released immediately after the injection in 60 1 aquaria and kept at 23°C. The mortality is checked in a daily interval.

### Dose optimisation in fish

The same experimental set-up as described under "RNAi-therapy in fish" is repeated three times, wherein instead of 15 µl of the 20 µM RNAi dilution, 15 µl of a 2 µM, a 200 µM and a 2 mM siRNA dilution are used.

### Prophylactic treatment in fish

The same experimental set-up as described under "RNAi-therapy in fish" is repeated, wherein the fish are not treated with the RNAi-therapy immediately after they received a virus injection, but 5 days before the virus is injected into the fish.

### Results

Cell survival differed significantly in our eight treatments (ANOVA: F_{7,16} = 32,293; p < 0.001). The non-infected controls (white bar in figure 1) survived significantly better than the KHV infected cell cultures (black bars in figure 1), whereas significance was tested with the Fisher Least Significant Difference Post Hoc Test. The siRNA-mix treatment with all four siRNA-fragments led to a clearly higher survival of KHV infected cell cultures compared to the infected, but untreated culture. The KHV infected cell cultures that were treated with the siRNA-mix showed a survival of 58% on a scale from 0 (untreated infected cell culture) to 1 (non-infected control).

We thus demonstrated for the first time that an RNAi-treatment can significantly increase the survival of KHV-infected cells.

## Claims

1. An RNAi agent, or a composition comprising at least one RNAi agent, or an expression vector capable of transcribing an RNAi agent, or a host cell comprising an expression vector capable of transcribing an RNAi agent for use in prophylactic or therapeutic treatment of freshwater fishes of the family *Cyprinidae.*

2. An RNAi agent, or composition, or expression vector, or host cell for use according to claim 1, wherein *Cyprinus carpio* (common carp), preferably the type Koi are prophylactically or therapeutically treated, and the disease is a Koi Herpes Virus (KHV) infection.

3. An RNAi agent for use according to claim 1 or 2, wherein the antisense strand is complementary to a target RNA transcribed of and/or originating from koi herpes virus (KHV) or contains not more than 1, 2 or 3 nucleotide mismatches to said target RNA within the duplex region.

4. An RNAi agent for use according to any of claims 1 to 3, wherein the antisense strand is complementary or contains not more than 1, 2 or 3 nucleotide mismatches to the RNA molecules transcribed of and/or originating from an KHV ORF with a sequence selected from any of SEQ ID No. 13 to 16 and ORF2 (GenBank ABG42833.1), ORF4 (GenBank ABG42835.1), ORF9 (GenBank ABG42840.1), ORF12 (GenBank ABG42843.1), ORF16 (GenBank ABG42847.1), ORF19 (GenBank ABG42850.1), ORF22 (GenBank ABG42853.1), ORF23 (GenBank ABG42854.1), ORF24 (GenBank ABG42855.1), ORF25 (GenBank ABG42856.1), ORF28 (GenBank ABG42857.1), ORF29 (GenBank ABG42858.1), ORF31 (GenBank ABG42859.1), ORF32 (GenBank ABG42860.1), ORF33 (GenBank ABG42861.1), ORF39 (GenBank ABG42867.1), ORF41 (GenBank ABG42868.1), ORF46 (GenBank ABG42873.1), ORF47 (GenBank ABG42874.1), ORF48 (GenBank ABG42875.1), ORF54 (GenBank ABG42881.1), ORF55 (GenBank ABG42882.1), ORF61 (GenBank ABG42888.1), ORF62 (GenBank ABG42889.1), ORF64 (GenBank ABG42891.1), ORF65 (GenBank ABG42892.1), ORF66 (GenBank ABG42893.1), ORF68 (GenBank ABG42895.1), ORF78 (GenBank ABG42905.1), ORF80 (GenBank ABG42907.1), ORF81 (GenBank ABG42908.1), ORF82 (GenBank ABG42909.1), ORF83 (GenBank ABG42910.1), ORF87 (GenBank ABG42914.1), ORF90 (GenBank ABG42917.1), ORF94 (GenBank ABG42921.1), ORF98 (GenBank ABG42925.1), ORF99 (GenBank ABG42926.1), ORF104 (GenBank ABG42931.1), ORF105 (GenBank ABG42932.1), ORF107 (GenBank ABG42934.1), ORF108 (GenBank ABG42935.1), ORF 112 (GenBank ABG42939.1), ORF 114 (GenBank ABG42941.1), ORF 115 (GenBank ABG42942.1), ORF 116 (GenBank ABG42943.1), ORF123 (GenBank ABG42950.1), ORF 124 (GenBank ABG42951.1), ORF125 (GenBank ABG42952.1), ORF126 (GenBank ABG42953.1), ORF128 (GenBank ABG42955.1), ORF131 (GenBank ABG42958.1), ORF134 (GenBank ABG42961.1), ORF136 (GenBank ABG42963.1), ORF137 (GenBank ABG42964.1), ORF138 (GenBank ABG42965.1), ORF139 (GenBank ABG42966.1)
ORF140 (GenBank ABG42967.1), ORF 141 (GenBank ABG42968.1), ORF144 (GenBank ABG42971.1), ORF146 (GenBank ABG42973.1), ORF148 (GenBank ABG42975.1), ORF149 (GenBank ABG42976.1), ORF150 (GenBank ABG42977.1), or ORF153 (GenBank ABG42980.1)
within the duplex region, preferably the ORF is selected from ORF 72 according to SEQ ID No. 13, ORF 71 according to SEQ ID No. 14, ORF 79 according to SEQ ID No. 15 and ORF 92 according to SEQ ID No. 16 of KHV.

5. An RNAi agent wherein the antisense strand is complementary to a target RNA transcribed of and/or originating from koi herpes virus (KHV) or contains not more than 1 nucleotide mismatch to said target RNA within the duplex region.

6. An RNAi agent wherein the antisense strand is complementary to a target RNA transcribed of and/or originating from koi herpes virus (KHV) or contains not more than 1, 2 or 3 nucleotide mismatches to the RNA molecules transcribed of and/or originating from an ORF selected from ORF 72 according to SEQ ID No. 13, ORF 71 according to SEQ ID No. 14, ORF 79 according to SEQ ID No. 15 and ORF 92 according to SEQ ID No. 16 of KHV within the duplex region.

7. An RNAi agent for use according to claims 1 to 4 or an RNAi agent according to claims 5 to 6, wherein the RNAi agent is selected from a siRNA or a shRNA molecule.

8. An RNAi agent for use according to claims 1 to 4 and 7 or an RNAi agent according to claims 5 to 6, wherein the RNAi agent comprises a sense strand and an antisense strand and wherein the antisense strand comprises a sequence of at least 16, 17, or 18 nucleotides

9. An RNAi agent for use according to claims 1 to 4 and 7, 8 or an RNAi agent according to claims 6 to 8 wherein the RNAi agent comprises a sense strand and an antisense strand and wherein the antisense strand is at least over contiguous 16 or 17 or 18 nucleotides complementary to a target RNA transcribed of and/or originating from an open reading frame of the KHV or wherein not more than 1, 2, or 3 mismatches occur within the duplex region.

10. An RNAi agent for use according to claims 1 to 4 and 7 to 9 or an RNAi agent according to claims 5 to 9, wherein the RNAi agent comprises a sense strand and an antisense strand and wherein
(i) the sense strand comprises a sequence of at least 16, 17, or 18 nucleotides and/or
(ii) the sense strand is over at least 16, 17, or 18 contiguous nucleotides complementary to the antisense strand or contains not more than 1, 2, or 3 nucleotide mismatches within the duplex region.

11. An RNAi agent, wherein
(i) the antisense strand comprises at least 15 contiguous nucleic acids of one of SEQ ID NO: 1 to 4 or the molecules of Table (II), or
(ii) the antisense strand is selected from one of SEQ ID NO: 1 to 4 or the molecules of Table (II), or
(iii) the antisense strand of the RNAi agent has at least 80%, preferably at least 90% sequence identity to an antisense strand of (i) or (ii) and/or contains 1, 2 or 3 mismatches to a target RNA transcribed of and/or originating from the genomic sequence of KHV,
wherein Table (II) is:
| Sequence (5'-3') | |
|---|---|
| UUAAAGUUGUGGAACUCGGCCdTdT | (SEQ ID No. 10) |
| UCUAGCUUGAACCUCACACa*c*dT*dT | |
| UAGUUGAAGACAAACAGCACGUCGCdTdT | (SEQ ID No. 12) |
| UCUCUCAGAAAGUCGUUGAGGUUGG*U*G | |
and wherein
(i) * marks phosphorothioate linkages
(ii) the lower case letters indicate 2'-O-methyl-modified nucleotides, and
(iii) dT means deoxythymidine

12. An expression vector capable of transcribing an RNAi agent according to any of claims 5 to 11 *in vivo* and/or *in vitro.*

13. A host cells comprising an expression vector according to claim 12.

14. A composition comprising at least one RNAi agent according to claims 5 to 11.

15. A composition according to claim 14, comprising 2, 3, 4, 5, 6 or more different RNAi agents.

16. A composition according to claim 14 or 15, comprising
(a) a mixture of RNA molecules, wherein the RNA molecules are **characterized by** the sequences according to SEQ ID No. 1 to 8, or
(b) a mixture of RNA molecules, wherein the RNA molecules are **characterized by** or comprise the sequences according to SEQ ID No. 1 to 8, which may optionally carry one or two overhangs and/or which may optionally contain a modified nucleotide and/or nucleotide linkage, or
(c) a mixture of the molecules, wherein the RNA molecules **characterized by** the sequences or chemical structures of Table (I):
| Sequence (5'-3') | |
|---|---|
| GGCCGAGUUCCACAACUUUAAdTdT | (SEQ ID No. 9) |
| UUAAAGUUGUGGAACUCGGCCdTdT | (SEQ ID No. 10) |
| GUGUGUGAGGUUCAAGCUAG*A*dT*dT | |
| UCUAGCUUGAACCUCACACa*c*dT*dT | |
| GCGACGUGCUGUUUGUCUUCAACUAdTdT | (SEQ ID No. 11) |
| UAGUUGAAGACAAACAGCACGUCGCdTdT | (SEQ ID No. 12) |
| CACCAACCUCAACGACUUUCUGAGA*G*A | |
| UCUCUCAGAAAGUCGUUGAGGUUGG*U*G | |
Wherein
(i) * marks phosphorothioate linkages
(ii) the lower case letters indicate 2'-O-methyl-modified nucleotides, and
(iii) dT means deoxythymidine

17. A fish comprising an RNAi agent, or composition, or expression vector, or a host cell according to any of claims 5 to 16.

18. The RNAi agents for use according to claims 1 to 4 and 7 to 10, wherein Poly(I:C), or Poly(A:U), or Poly(G:C), or combinations thereof is/are in addition administered separately or together with the RNAi agents.
